# DEMANDE DE BREVET EUROPEEN

(11) **EP 0 699 679 A1**
(43) Date de publication de la demande: **06.03.1996**
(21) Numéro de dépôt: 95401971.7
(22) Date de dépôt: 30.08.1995
(51) Int. Cl.: C07D 493/10, C07H 7/06, C07H 7/02, C07H 15/10, C07H 15/26, C07H 9/04, C07D 309/30, C07D 407/04, C07D 407/12, A61K 31/70, A61K 31/35

(54) **Nouveaux composés tétrahydropyraniques, leur procédé de préparation et les compositions pharmaceutiques qui les contiennent**

(30) Priorité: 31.08.1994 FR 9410462
(71) Demandeur: ADIR ET COMPAGNIE, F-92415 Courbevoie Cédex (FR)
(72) Inventeur: Billington, David, Solihull, West Midlands B90 3RR (GB); Dorey, Gilbert, F-92370 Chaville (FR); Leon, Pascale, F-78280 Guyancourt (FR); Atassi, Ghanem, F-92400 Saint Cloud (FR); Pierre, Alain, F-78580 Les Alluets-Le-Roi (FR); Burbridge, Michael, F-92400 Courbevoie (FR); Guilbaud, Nicolas, F-75015 Paris (FR)

(57) **Abrégé**

Composés de formule (I) : 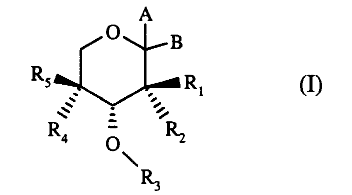 dans laquelle :
- A: - soit représente -OR₆ et B représente -CH₂-X,
- soit forme avec B et l'atome de carbone qui les porte un hétérocycle oxygéné choisi parmi l'oxirane, le 2,2-diméthyl[1,3]dioxolane, et la [1,3]dioxolan-2-one,
et R₁, R₂, R₃, R₄, R₅ R₆ et X sont tels que définis dans la description.

Médicaments.

## Description

La présente invention concerne de nouveaux composés glucidiques possédant une structure tétrahydropyranique, leur procédé de préparation et les compositions pharmaceutiques qui les contiennent. Les composés de la présente invention trouvent une utilisation thérapeutique tout à fait intéressante grâce à leur pouvoir inhibiteur d'angiogenèse.

L'angiogenèse (ou néovascularisation) est définie comme le développement et la croissance de nouveaux vaisseaux sanguins capillaires. Le processus d'angiogenèse est essentiel dans de nombreuses situations physiologiques dont le développement de l'embryon, la cicatrisation normale de blessures et le développement de l'endometrium après menstruation. En dehors de ces situations, l'angiogenèse chez l'adulte normal est très rare et la mitose des cellules endothéliales qui génère les parois des vaisseaux sanguins est très lente, avec des temps de renouvellement cellulaire mesurés en années.

Une angiogenèse anormale (c'est-à-dire la stimulation de la croissance de nouveaux vaisseaux sanguins due à un syndrome pathologique) est une caractéristique établie pour de nombreuses maladies, notamment la rétinopathie diabétique, l'arthrite rhumatoïde, les hémangiomes et la croissance de tumeurs solides. L'angiogenèse peut également jouer un rôle important dans d'autres maladies comme la maladie artério-coronaire.

Dans le domaine de l'oncologie, il a été montré que la croissance de tumeurs solides est tout à fait dépendante du développement constant de nouveaux vaisseaux sanguins et qu'il est corrélé, pour les métastases de certains cancers, avec la taille croissante de la tumeur primaire. (J. Folkman, *New Engl. Med.*, *285* (1974), 1182-1185).

Un traitement pharmaceutique (c'est-à-dire à l'aide d'un inhibiteur d'angiogenèse) peut donc stopper la croissance de tumeurs primaires, empêcher ou réduire la formation de métastases, empêcher l'apparition de croissances secondaires. De tels inhibiteurs d'angiogenèse sont également utiles dans le traitement de maladies non-néoplasiques mentionnées précédemment dans lesquelles apparaît une activité angiogénique.

Les besoins de la thérapeutique exigent le développement constant de nouveaux composés inhibiteurs d'angiogenèse dans le but d'obtenir des principes actifs à la fois plus actifs, plus spécifiques et moins toxiques.

La présente invention concerne de nouveaux composés possédant une structure tétrahydropyranique et présentant une originalité structurale et pharmacologique par rapport aux composés décrits dans l'art antérieur.

Plus particulièrement, la présente invention a pour objet des composés de formule générale (I): 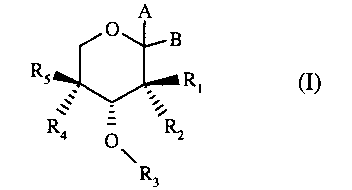 dans laquelle :
- **A**: - soit représente -OR₆ et **B** représente -CH₂-X,
- soit forme avec **B** et l'atome de carbone qui les porte un hétérocycle oxygéné choisi parmi l'oxirane, le 2,2-diméthyl[1,3]dioxolane, et la [1,3]dioxolan-2-one,
- **R₁**: - soit représente le radical : 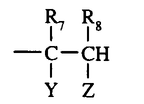 dans lequel Y et Z, soit représentent chacun l'hydrogène, soit forment ensemble une double liaison, ou forment ensemble, avec les atomes de carbone qui les portent, un cycle oxirane,
   et **R₂** est choisi parmi l'hydrogène, le radical hydroxy et le radical -OR₉,
- soit le radical : 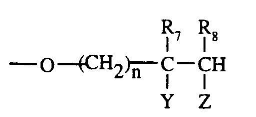 dans lequel n prend les valeurs entières 1 à 4, bornes incluses, et Y et Z, soit représentent chacun l'hydrogène, soit forment ensemble une double liaison, ou forment ensemble, avec les atomes de carbone qui les portent, un cycle oxirane,
   et **R₂** représente l'hydrogène,
- soit **R₁** représente l'hydrogène,
   et **R₂** représente le radical : 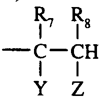 tel que défini ci-dessus,
- **R₃**: est choisi parmi l'hydrogène, un radical alkyle linéaire ou ramifié comportant de 1 à 6 atomes de carbone et éventuellement substitué, le radical benzyle éventuellement substitué, le radical benzoyle éventuellement substitué, le radical formyle éventuellement substitué, un radical acyle linéaire ou ramifié, éventuellement insaturé, comportant de 1 à 6 atomes de carbone et éventuellement substitué, le radical allyle, et le radical carbamoyle éventuellement mono- ou di-substitué,
- **R₄**: - soit est choisi parmi le radical hydroxy, un radical alkoxy linéaire ou ramifié comportant de 1 à 6 atomes de carbone et éventuellement substitué, le radical carbamoyloxy éventuellement mono- ou di-substitué, le radical pipérazinylcarbonyloxy substitué en position 4 par le radical R₉ et le radical imidazol-1-yl-carbonyloxy,
   et **R₅** représente l'hydrogène,
- soit forme avec **R₅** et l'atome de carbone qui les porte un groupement carbonyle,
- **R₆, R₇** et **R₈**: sont choisis indépendamment l'un de l'autre parmi l'hydrogène, un radical alkyle linéaire ou ramifié comportant de 1 à 6 atomes de carbone et éventuellement substitué, et un radical phénylalkyle dans lequel le groupement phényle est éventuellement substitué et le groupement alkyle, linéaire ou ramifié et éventuellement substitué, comporte de 1 à 6 atomes de carbone,
- **R₉**: est choisi parmi l'hydrogène, un radical alkyle linéaire ou ramifié comportant de 1 à 6 atomes de carbone et éventuellement substitué, le radical formyle éventuellement substitué, un radical acyle linéaire ou ramifié, éventuellement insaturé, comportant de 1 à 6 atomes de carbone et éventuellement substitué, un radical alkoxycarbonyle linéaire ou ramifié comportant de 1 à 6 atomes de carbone et éventuellement substitué, le radical benzyle éventuellement substitué, et le radical carbamoyle éventuellement mono- ou di-substitué,
- **X**: est choisi parmi le radical hydroxy, un atome d'halogène, un radical phénylsulfonyloxy éventuellement substitué et un radical alkylsulfonyloxy linéaire ou ramifié, éventuellement substitué et comportant de 1 à 6 atomes de carbone,
leurs éventuels isomères optiques et géométriques sous forme pure ou en mélange, ainsi que leurs éventuels sels d'addition à un acide, pharmaceutiquement acceptables,
étant entendu que :
- le terme "éventuellement mono- ou di-substitué" associé aux radicaux carbamoyle et carbamoyloxy ci-dessus définis signifie que un seul ou les deux atomes d'hydrogène portés par l'atome d'azote peut ou peuvent être substitués (et de manière indépendante, lorsque les deux atomes d'hydrogène sont substitués) par:
   - un radical alkyle linéaire ou ramifié comportant de 1 à 6 atomes de carbone, et éventuellement substitué,
   - le radical formyle éventuellement substitué,
   - un radical acyle linéaire ou ramifié, comportant de 1 à 6 atomes de carbone, éventuellement insaturé et éventuellement substitué,
   - le radical benzoyle, éventuellement substitué,
   - le radical phényle, éventuellement substitué,
   - le radical naphtyle, éventuellement substitué, et
   - un radical amino éventuellement substitué par un ou deux radicaux alkyles comportant de 1 à 6 atomes de carbone en chaîne linéaire ou ramifiée et chacun d'eux pouvant être éventuellement substitués,
- le terme "éventuellement substitué" associé aux radicaux alkyle, alkoxy, alkoxycarbonyle, formyle, acyle, benzyle, benzoyle, phényle et naphtyle signifie que ces radicaux peuvent être substitués par une ou plusieurs entités chimiques choisies parmi hydroxy, halogène, trihalogénométhyle, amino, alkylamino, dialkylamino, alkoxy linéaire ou ramifié comportant de 1 à 6 atomes de carbone, alkoxycarbonyle linéaire ou ramifié comportant de 1 à 6 atomes de carbone et acyle linéaire ou ramifié comportant de 1 à 6 atomes de carbone,
- le terme éventuellement substitué associé aux radicaux alkylsulfonyloxy et phénylsulfonyloxy signifie que ces radicaux peuvent être substitués par un ou plusieurs radicaux alkyles linéaires ou ramifiés et comportant de 1 à 6 atomes de carbone,
- par radical acyle insaturé, on entend plus particulièrement les radicaux acryloyle et méthacryloyle.

Dans la présente invention, est entendu par radical carbamoyle, le radical 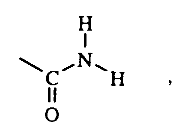 et par radical carbamoyloxy, le radical

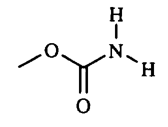

Parmi les acides utilisables pour la formation des sels pharmaceutiquement acceptables, on peut mentionner, à titre d'exemples non limitatifs, les acides chlorhydrique, phosphorique, sulfurique, tartrique, citrique, maléique, fumarique...

La présente invention s'étend également au procédé de préparation des composés de formule (I) caractérisé en ce que l'on soumet le 1,2:4,5-di-O-isopropylidène-β-_{D}-fructopyranose de formule (II) : 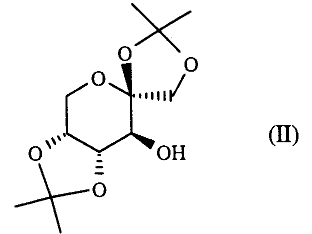 préparé selon le mode opératoire décrit par E.J. Prisbe *et al*. (*J. Org. Chem*, *41*, (1976), 1836-1846).
**soit** : à un oxydant, tel que le dichromate de pyridinium, de manière à obtenir la cétone de formule (III) : 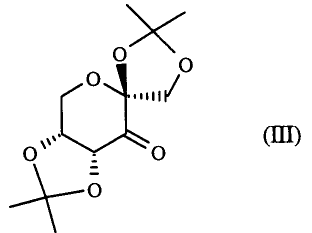 qui est soumise à l'action d'un composé de formule (IVa) préparé à partir de l'halogénure vinylique correspondant : 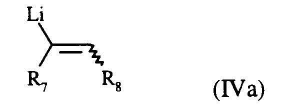 dans laquelle R₇ et R₈ sont choisis indépendamment l'un de l'autre parmi l'hydrogène, un radical alkyle linéaire ou ramifié comportant de 1 à 6 atomes de carbone, et éventuellement substitué, et un radical phénylalkyle dans lequel le groupement phényle est éventuellement substitué et le groupement alkyle, linéaire ou ramifié et éventuellement substitué, comporte de 1 à 6 atomes de carbone,
afin d'obtenir le composé de formule (Va₁) : 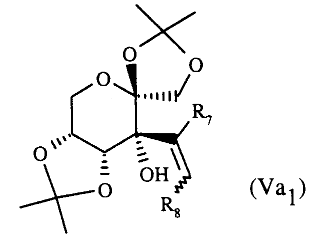 dans laquelle R₇ et R₈ sont tels que définis précédemment,
qui peut être éventuellement soumis à l'action d'un halogénure d'alkyle, d'un halogénure d'acyle, d'un halogénure de benzyle, d'un halogénoformiate d'alkyle, ou d'un isocyanate afin de conduire au composé de formule (Va₂) : 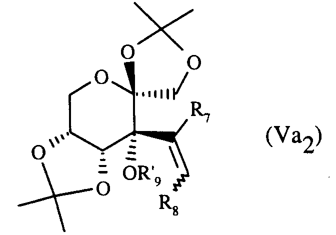 dans laquelle R₇ et R₈ sont tels que définis précédemment et R'₉ est choisi parmi un radical alkyle linéaire ou ramifié contenant de 1 à 6 atomes de carbone et éventuellement substitué, le radical formyle éventuellement substitué, un radical acyle contenant de 1 à 6 atomes de carbone et éventuellement substitué, le radical benzyle éventuellement substitué, un radical alkoxycarbonyle linéaire ou ramifié contenant de 1 à 6 atomes de carbone et éventuellement substitué, et un radical carbamoyle éventuellement mono- ou di-substitué,
composé de formule (Va₁) qui peut éventuellement être soumis à l'action de chlorure de méthyloxalyle, en présence de n-butyllithium, de façon à obtenir le composé de formule (Va₃) : 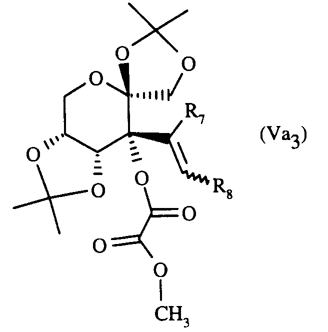 dans laquelle R₇ et R₈ sont tels que définis précédemment,
qui est ensuite soumis à l'action d'hydrure de tributylétain pour conduire au composé de formule (Va₄) : 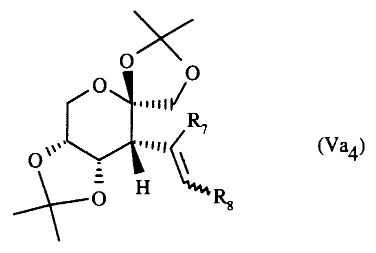 dans laquelle R₇ et R₈ sont tels que définis précédemment,
l'ensemble des composés de formule (Va₁) et (Va₂) formant le composé de formule (Va) : 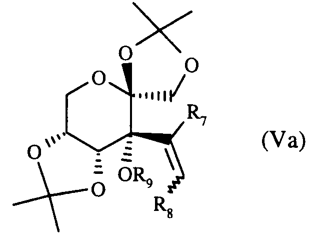 dans laquelle R₇ et R₈ sont tels que définis précédemment et R₉ est choisi parmi l'hydrogène et le radical R'₉ tel que défini précédemment,
**soit** : directement à l'action d'un composé de formule (IVb) : 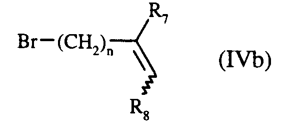 dans laquelle R₇ et R₈ sont tels que définis précédemment, et n prend les valeurs entières 1 à 4, bornes incluses,
afin d'obtenir le composé de formule (Vb) : 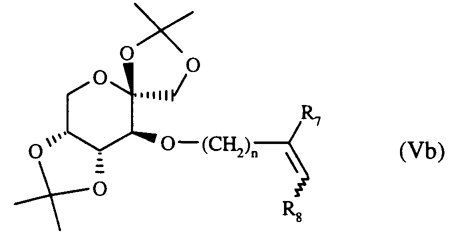 dans laquelle R₇, R₈ et n sont tels que définis précédemment,
les composés de formules (Va₄), (Va) et (Vb) étant ensuite hydrolysés, en milieu acide, par exemple l'acide acétique, pour conduire respectivement aux diols de formule (VIa₄), (VIa) et (VIb) : 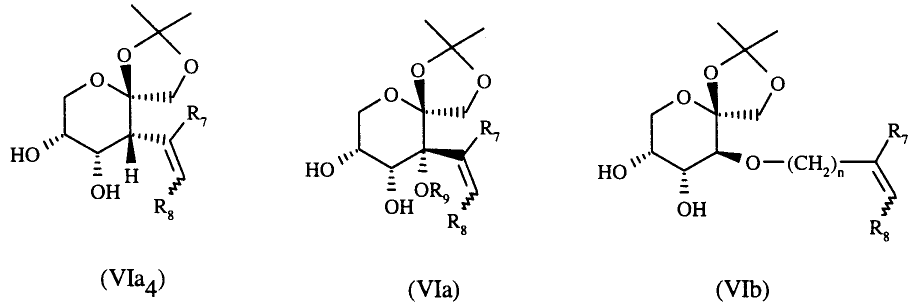 dans lesquelles R₇, R₈, R₉ et n sont tels que définis précédemment,
composés de formules (VIa₄), (VIa) et (VIb) qui peuvent être régiosélectivement substitués en présence d'oxyde de dibutylétain de manière à obtenir respectivement les composés de formules (VIIa4), (VIIa) et (VIIb) : 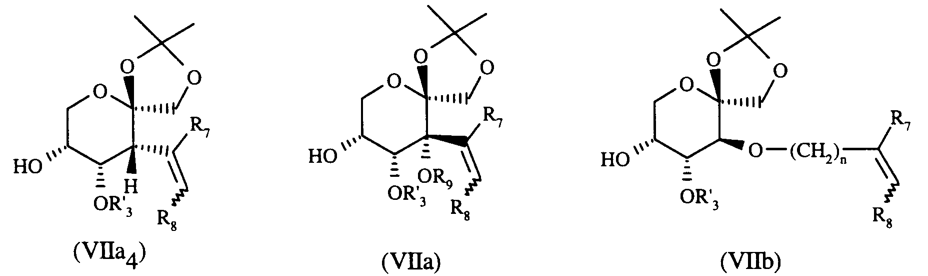 dans lesquelles R₇, R₈, R₉ et n sont tels que définis précédemment et R'₃ est choisi parmi un radical alkyle linéaire ou ramifié comportant de 1 à 6 atomes de carbone et éventuellement substitué, le radical benzyle éventuellement substitué, le radical benzoyle éventuellement substitué, le radical formyle éventuellement substitué, un radical acyle, éventuellement insaturé, linéaire ou ramifié comportant de 1 à 6 atomes de carbone et éventuellement substitué, le radical allyle, et le radical carbamoyle éventuellement mono- ou di-substitué,
l'ensemble des composés de formules (VIa₄) et (VIIa₄), (VIa) et (VIIa) et (VIb) et (VIIb) pouvant éventuellement être soumis à un réactif d'époxydation tel que l'acide 3-chloroperbenzoïque pour conduire respectivement aux composés de formules (VIIIa₄), (VIIIa) et (VIIIb) : 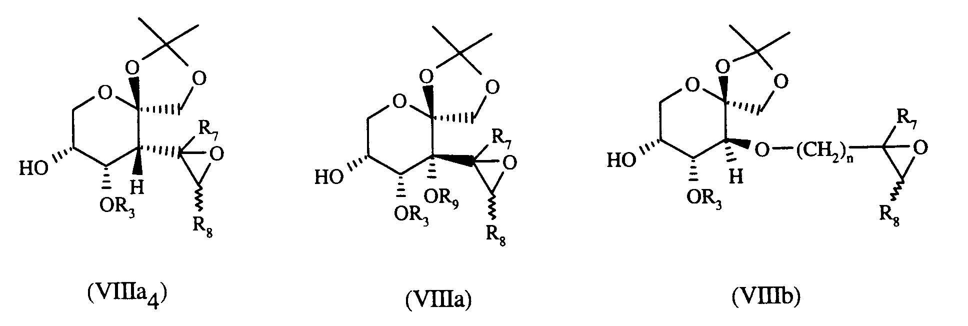 dans lesquelles R₇, R₈, R₉ et n sont tels que définis précédemment, et R₃ est choisi parmi l'hydrogène et le radical R'₃ tel que défini précédemment,
ou bien soumis à hydrogénation catalytique, afin d'obtenir respectivement les composés de formules (VIII'a₄), (VIII'a) et (VIII'b) : 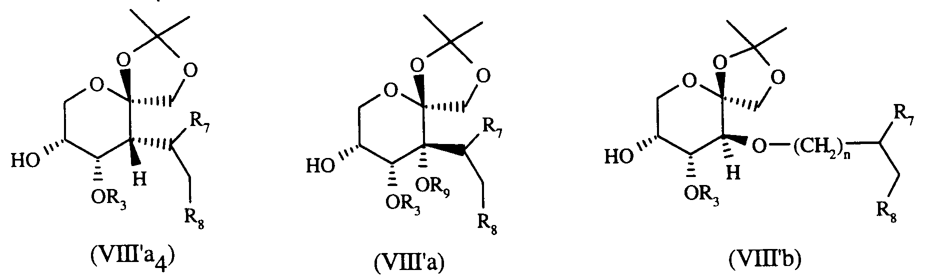 dans lesquelles R₃, R₇, R₈, R₉ et n sont tels que définis précédemment,
l'ensemble des composés de formules (VIa₄), (VIa), (VIb), (VIIa₄), (VIIa), (VIIb), (VIIIa₄), (VIIIa), (VIIIb), (VIII'a₄), (VIII'a) et (VIII'b) formant le composé de formule (VIII) : 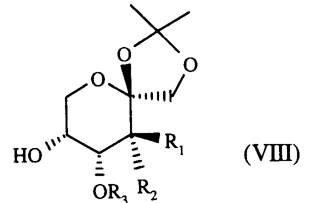 dans laquelle :
- R₁: - soit représente le radical : 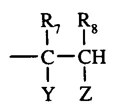 dans lequel Y et Z, soit représentent chacun l'hydrogène, soit forment ensemble une double liaison, soit forment ensemble, avec les atomes de carbone qui les portent, un cycle oxirane, et R₇ et R₈ sont tels que définis précédemment,
   et R₂ est choisi parmi l'hydrogène, le radical hydroxy et le radical -OR₉,
- soit le radical : 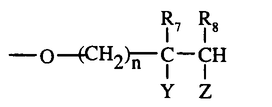 dans lequel n prend les valeurs entières 1 à 4, bornes incluses, et R₇, R₈, Y et Z sont tels que définis précédemment,
   et R₂ représente l'hydrogène,
- soit R₁ représente l'hydrogène,
   et R₂ représente le radical: 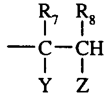 tel que défini ci-dessus,
   et R₃ est tel que précédemment défini, le composé de formule (VIII) étant ensuite éventuellement soumis :

- à l'action d'un agent alkylant dans des conditions classiques, par exemple après formation d'un anion à l'aide d'hydrure de sodium,
- à l'action d'un dérivé isocyanique en présence ou non d'un activateur tel que la 4-diméthylaminopyrine,
- où à l'action d'un agent carbonylant, le carbonyle diimidazole, conduisant au composé de formule (IXa) : 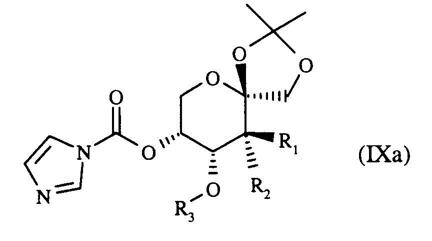 dans laquelle R₁, R₂ et R₃ sont tels que définis précédemment,
   lui-même conduisant, sous l'action d'une pipérazine substituée en position 4 par le radical R₉, au composé de formule (IXb) : 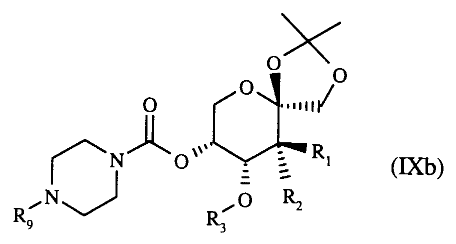 dans laquelle R₁, R₂, R₃ et R₉ sont tels que définis précédemment,
   l'ensemble des composés de formules (VIII), (IXa) et (IXb) formant le composé de formule (IX) : 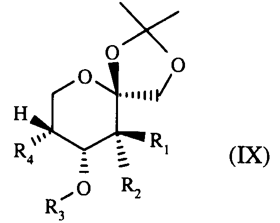 dans laquelle R₁, R₂ et R₃ sont tels que définis précédemment et R₄ est choisi parmi le radical hydroxy, un radical alkoxy linéaire ou ramifié comportant de 1 à 6 atomes de carbone et éventuellement substitué, le radical carbamoyloxy éventuellement mono- ou di-substitué, le radical pipérazinylcarbonyloxy substitué en position 4 par le radical R₉, et le radical imidazolylcarbonyloxy,
   le composé de formule (VIII) pouvant également être soumis à l'action d'un agent oxydant dans les conditions utilisées pour l'obtention du composé de formule (III), de manière à obtenir le composé de formule (X) : 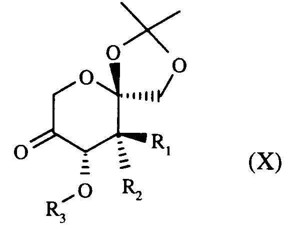 dans laquelle R₁, R₂ et R₃ sont tels que définis précédemment,
   l'ensemble des composés de formules (IX) et (X) formant le composé de formule (XI) : 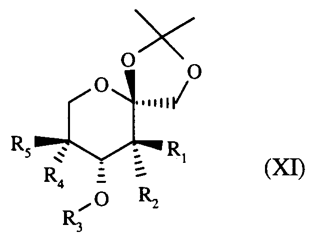 dans laquelle R₁, R₂ et R₃, sont tels que définis précédemment, et R₄ et R₅ sont tels que définis pour la formule (I),
qui peut être :
- **soit** : traité selon des méthodes classiques d'alcoolyse pour former le composé de formule (XII) : 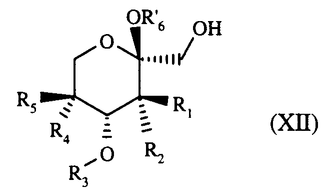 dans laquelle R₁, R₂, R₃, R₄ et R₅ sont tels que définis précédemment et R'₆ est choisi parmi un radical alkyle linéaire ou ramifié comportant de 1 à 6 atomes de carbone et éventuellement substitué, et un radical phénylalkyle dans lequel le groupement phényle est éventuellement substitué et le groupement alkyle, linéaire ou ramifié et éventuellement substitué, comporte de 1 à 6 atomes de carbone,
   puis soumis à des réactions classiques de substitution de façon à obtenir le composé de formule (XIII) : 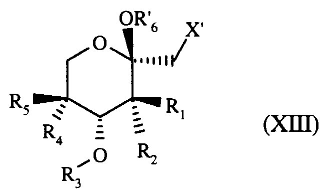 dans laquelle R₁, R₂, R₃, R₄, R₅ et R'₆ sont tels que définis précédemment et X' est choisi parmi un atome d'halogène, un radical phénylsulfonyloxy éventuellement substitué et un radical alkylsulfonyloxy linéaire ou ramifié, éventuellement substitué et comportant de 1 à 6 atomes de carbone,
- **soit** : hydrolysé, sous l'action d'une résine acide par exemple, en diol de formule (XIV) : 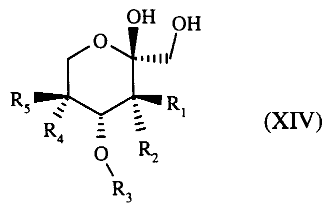 dans laquelle R₁, R₂, R₃, R₄ et R₅ sont tels que définis précédemment,
   puis éventuellement soumis à des réactions classiques de substitution de façon à obtenir le composé de formule (XV) : 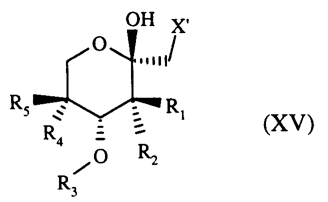 dans laquelle R₁, R₂, R₃, R₄, R₅ et X' sont tels que définis précédemment,
   composé de formule (XV) qui, lorsque X' représente l'atome d'iode, peut être transformé, sous l'action d'oxvde d'argent, en époxyde de formule (XVI) : 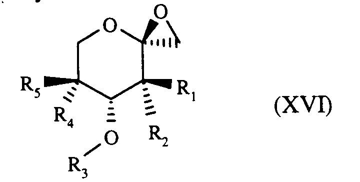 dans laquelle R₁, R₂, R₃, R₄, et R₅ sont tels que définis précédemment,
   le composé de formule (XIV) pouvant également être soumis à l'action de N,N-carbonyldiimidazole pour conduire au composé de formule (XVII) : 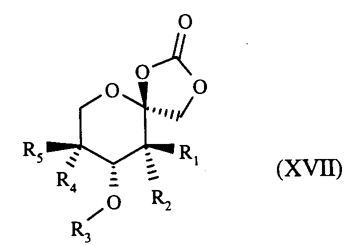 dans laquelle R₁, R₂, R₃, R₄, et R₅ sont tels que définis précédemment,
l'ensemble des composés de formules (XI), (XII), (XIII), (XIV), (XV), (XVI) et (XVII) formant l'ensemble des composés de formule (I) que l'on purifie le cas échéant par une technique classique de purification et dont on sépare, si on le souhaite, les isomères optiques et géométriques par une technique classique de séparation, et que l'on transforme éventuellement en leurs sels d'addition à un acide, pharmaceutiquement acceptables.

Le composé de formule (Va₁) dans lequel R₇ représente l'hydrogène peut avantageusement être obtenu en faisant réagir un organolithien, préparé à partir du dérivé propargylique correspondant, de formule (IVa') : 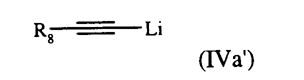 dans laquelle R₈ est tel que défini précédemment,
puis par hydrogénation catalytique de l'alcyne obtenu de formule (Va₁') : 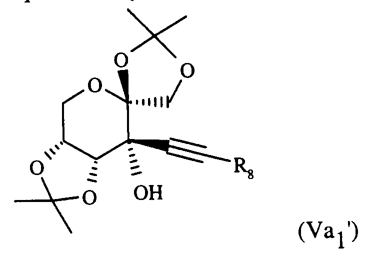 dans laquelle R₈ est tel que défini précédemment.

Le composé de formule (VIII) peut être protégé régiosélectivement en un dérivé silylé de formule (VIII') : 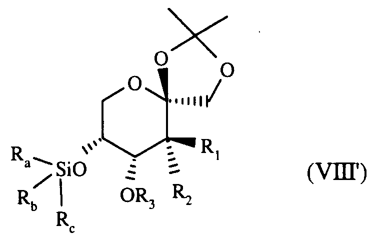 dans laquelle R₁ et R₂ sont tels que définis précédemment et Rₐ, R_{b} et R_{c} représentent indépendamment l'un de l'autre un radical alkyle linéaire ou ramifié contenant de 1 à 6 atomes de carbone ou un radical phényle.

De manière générale, les protections et déprotections des radicaux hydroxy portés par les dérivés pyranosiques, les réactions d'hydrogénation catalytique, de même que les réactions d'époxydation au moyen d'acide 3-chloroperbenzoïque, peuvent être effectuées au moment jugé opportun par l'homme de l'art, au cours de la synthèse de chaque composé.

Les composés de formule (I) possèdent d'intéressantes propriétés pharmacologiques. En effet, ces composés sont de puissants inhibiteurs d'angiogenèse qui ont l'avantage de présenter, par rapport aux composés de référence, une toxicité beaucoup moins importante. lls présentent donc un index thérapeutique excellent. Ces composés trouvent ainsi une application en thérapeutique en tant qu'agents anti-tumoraux, dans l'inhibition de la formation et de la croissance des métastases, ainsi que dans le traitement de la rétinopathie diabétique, de l'arthrite rhumatoïde, des hémangiomes et des maladies artério-coronaires, et plus généralement dans les affections dues ou reliées aux troubles de l'angiogenèse.

La présente invention a également pour objet les compositions pharmaceutiques contenant les composés de formule (I), leurs éventuels stéréoisomères ou leurs éventuels sels d'addition à un acide pharmaceutiquement acceptables, seuls ou en combinaison avec un ou plusieurs excipients ou véhicules inertes et non toxiques.

Parmi les compositions pharmaceutiques selon l'invention, il sera cité plus particulièrement celles qui conviennent pour l'administration orale, parentérale, nasale, rectale, perlinguale, oculaire ou respiratoire, et notamment les comprimés simples ou dragéifiés, les comprimés sublinguaux, les sachets, paquets, gélules, glossettes, les tablettes, les suppositoires, les crèmes, les pommades, les gels dermiques, les préparations injectables ou buvables, les aérosols, les gouttes oculaires ou nasales.

La posologie utile varie selon l'âge et le poids du patient, la voie d'administration, la nature de l'indication thérapeutique et des traitements éventuels associés et s'échelonne entre 0,01 et 1 g par jour, en une ou plusieurs administrations.
Les exemples suivants illustrent l'invention mais ne la limitent en aucune façon. Les produits de départ sont connus ou préparés à partir de modes opératoires connus.

La nomenclature utilisée pour la dénomination des composés de l'invention est celle préconisée dans "Modern Carbohydrate Chemistry", de Roger W. Binkley, Editions Marcel Dekker Inc., New York (1988).

La numérotation et la stéréochimie du β-_{D}-psicopyranose employées dans les exemples suivants sont conformes à la formule :

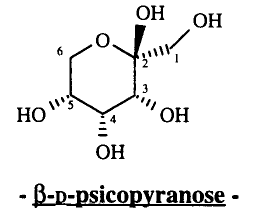

La numérotation et la stéréochimie du β-_{D}-fructopyranose employées dans les exemples suivants sont conformes à la formule :

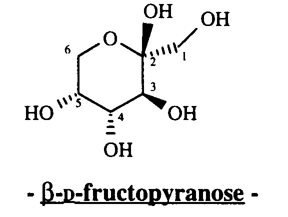

### Exemple 1: 1,2-O-Isopropylidène-3-isopropényl-β-_{D}-psicopyranose

### Etape A : 1,2:4,5-di-O-isopropylidène-β-_{D}-érythro-2,3-hexodiulo-2,6-pyranose

A 1,00 g (3,84 mmoles) de 1,2:4,5-di-O-isopropylidène-β-_{D}-fructopyranose, préparé suivant le mode opératoire décrit par Prisbe E. J. *et al.* (*J. Org. Chem.*, *41*, (1976), 1836), dissous dans 20 ml de dichlorométhane anhydre sont ajoutés 2,44 g de tamis moléculaire 0,3 nm. Le milieu réactionnel est refroidi à 10°C à l'aide d'un bain de glace et 1.38 g (6,48 mmoles) de dichromate de pyridinium suivis de 0,3 ml (5,24 mmoles) d'acide acétique glacial sont ajoutés. Le mélange réactionnel est placé sous agitation pendant 5 heures à 10°C puis concentré (jusqu'à 5 ml) sous vide. 30 ml d'éther diéthylique sont ajoutés et l'ensemble est filtré . Le filtrat est alors évaporé et le résidu solide résultant est chromatographié sur gel de silice (éluant : acétate d'éthyle/pentane, 3:2). 0,81 g (3,14 mmoles) de produit attendu sous la forme d'un solide de couleur blanche sont isolés.
Rendement : 81%
Caractéristiques spectrales :
¹H RMN (DMSO) δ (ppm) : 1,30 (3H, s) ; 1,31 (3H, s) 1,35 (3H, s) ; 1,48 (3H, s) ; 3,90 (1H, d) ; 4,05 (1H, d) ; 4,25 (1H, d) ; 4,40 (1H, d) ;4,61 (1H, dd) 4,82 (1H, d).

### Etape B : 1,2:4,5-di-O-isopropylidène-3-isopropényl-β-_{D}-psicopyranose

A une solution de 1,03 ml 2-bromopropène (1,40 g ; 11,59 mmoles) dans 8,5 ml de diéthyléther anhydre refroidie à -78°C, sont ajoutés goutte-à-goutte et sous atmosphère d'azote 5,69 ml de tert-butyllithium (1,7 M dans le pentane soit 9,67 mmoles). L'ensemble est laissé sous agitation à -78°C pendant 5 minutes puis à 0°C pendant 1 heure. Cette solution est alors additionnée goutte-à-goutte à une solution du produit obtenu à l'Etape A (1 g ; 3,87 mmoles) dans 15 ml de toluène anhydre refroidie à -78°C. Après 45 minutes d'agitation à -78°C le mélange réactionnel est versé dans une solution aqueuse à 10% de chlorure d'ammonium (40 ml) refroidie à 0°C. L'ensemble est dilué à l'éther diéthylique (100 ml) est le mélange réactionnel est extrait. Le traitement habituel de la phase organique fournit un résidu huileux qui est chromatographié sur gel de silice (éluant : pentane/acétate d'éthyle, 8:1). 0,55 g (1,83 mmoles) de produit attendu sont obtenus sous forme d'une mousse blanche.
Rendement : 47%
Caractéristiques spectrales :
¹H RMN (DMSO) δ (ppm) : de 1,25 à 1,50 (12H, m) ; 1,85 (3H, s) ; 3,40 (1H, s, échangeable par D₂O) ; 3,80 (1H, d) ; 4,00 (1H, d) ; 4,05 (2H, d) ; 4,34 (1H, dd) ; 4,45 (1H, d) ; 5,10 (1H, s) ; 5,25 (1H, s).

### Etape C : 1,2-O-isopropylidène-3-isopropényl-β-_{D}-psicopyranose

Une solution de 4 g (13,32 mmoles) de composé obtenu à l'Etape B dans 100 ml d'un mélange acide acétique/eau, 4:1 est placée sous agitation à température ambiante pendant 17 heures. Les solvants sont évaporés sous pression réduite et le résidu repris dans 50 ml de toluène puis évaporé à nouveau. Cette opération est répétée 2 fois pour conduire après 24 heures de séchage sous vide poussé à 3.28 g (12,60 mmoles) de composé attendu (pureté >99% par chromatograhie en phase gazeuse) sous la forme d'une poudre de couleur blanche.
Rendement : 96%
Caractéristiques spectrales :
¹H RMN (DMSO) δ (ppm) : 1,30 (3H, s) ; 1,38 (3H, s) ; 1,85 (3H, s) ; 3,65 (1H, d) ; 3,75 (1H, d) ; 3,81 (1H, m) ; 3,82 (1H, m) ; 3,85 (1H, d) ; 4,10 (1H, d) ; 4,55 (1H, d, échangeable par D₂O) ; 5,05 (1H, s) ; 5.08 (1H, s, échangeable par D₂O) ; 5,16 (1H, s) ; 5,60 (1H, d, échangeable par D₂O).

### Exemple 2 : 1,2-O-Isopropylidène-3-isopropényl-4-O-méthyl-β-_{D}-psicopyranose

A une solution de 4 g (15,37 mmoles) du composé obtenu à l'Exemple 1 dans 120 ml de méthanol sont ajoutés 7,65 g (30,70 mmoles) d'oxyde de dibutylétain. Le mélange réactionnel hétérogène est placé sous agitation et chauffé à reflux jusqu'à obtention d'une solution limpide (soit environ 24 heures). Le mélange est refroidi puis le solvant évaporé sous pression réduite. Le résidu solide est repris dans 70 ml de dioxane et placé sous agitation et atmosphère d'azote. 6 ml (96.37 mmoles) d'iodure de méthyle sont ajoutés et l'ensemble est chauffé à reflux jusqu'à disparition complète du produit de départ (soit environ 60 heures). Le dioxane est alors évaporé sous vide et le résidu solide résultant est chromatographié sur gel de silice (éluant : pentane/acétate d'éthyle, 2:1 puis 1:1). 3,8 g (13,85 mmoles) de composé attendu sous la forme d'un solide blanc sont obtenus.
Rendement : 90%
Caractéristiques spectrales :
¹H RMN (DMSO) δ (ppm) : 1,25 (3H, s) ; 1,32 (3H, s) ; 1,80 (3H, s) ; 3,28 (3H, s) ; 3,50 (1H, d) ; 3,70 (2H, d) ; 3,82 (1H, d) ; 4,08 (1H, d) ; 4,18 (1H, s élargi) ; 5,02 (1H, s) ; 5,15 (1H, s) ; 5,38 (1H, s , échangeable par D₂O) ; 5,70 (1H, s élargi, échangeable par D₂O).

### Exemple 3: 5-O-Chloroacétylcarbamoyl-1,2-O-Isopropylidène-3-isopropényl-4-O-méthyl-β-_{D}-psicopyranose

A une solution de 5,00 g (18,23 mmoles) du composé obtenu à l'Exemple 2 dans 90 ml de dichlorométhane anhydre refroidie à 0°C sont ajoutés, goutte-à-goutte et sous atmosphère d'azote, 2,39 ml (3,35 g ; 28,05 mmoles) d'isocyanate de chloroacétyle. Après 40 minutes d'agitation à 0°C le mélange réactionnel est versé dans 50 ml d'eau glacée et l'ensemble est laissé sous agitation pendant 1 heure. Après traitement habituel de la phase organique et purification du produit brut par chromatographie sur gel de silice (éluant : éther diéthylique/ éther de pétrole, 4:1), 5,7 g (14,47 mmoles) du composé attendu sous la forme d'une mousse de couleur blanche sont obtenus.
Rendement : 79%
Caractéristiques spectrales :
¹H RMN (DMSO) δ (ppm) : 1,30 (3H, s) ; 1,40 (3H, s) ; 1,83 (3H, s) ; 3,29 (3H, s) ; 3,75 à 3,90 (3H, m) ; 4,00 (1H, d) ; 4,10 (1H, s, échangeable par D₂O) ; 4,12 (1H, d) ; 4,46 (2H, s) ; 5,09 (1H, s élargi) ; 5,29 (1H, d) ; 5,41 (1H, s) ; 11,02 (1H, s, échangeable par D₂O).

| Analyse élémentaire : (Formule brute : C₁₆H₂₄ClNO₈ M=393,82) | | | | |
|---|---|---|---|---|
| | **C** | **H** | **N** | **Cl** |
| **% trouvé** | 48,78 | 5,80 | 3,82 | 9,03 |
| **% calculé** | 48,80 | 6,14 | 3,56 | 9,00 |

### Exemple 4: 5-O-Chloroacétylcarbamoyl-1,2-O-isopropylidène-3-(2-méthyloxiran-2-yl)-4-O-méthyl-β-_{D}-psicopyranose

A une solution de 5,70 g (14,47 mmoles) du composé obtenu à l'Exemple 3 dans 140 ml de dichlorométhane sont ajoutés 5,00 g (21,15 mmoles) d'acide 3-chloroperbenzoïque à 72%. Le mélange réactionnel est placé sous agitation à température ambiante pendant 15 heures. Le solide formé en cours de réaction est filtré puis le filtrat évaporé sous pression réduite. Le résidu solide est purifié par chromatographie sur gel de silice (éluant : acétate d'éthyle/ pentane, 2:1). Le composé attendu (4,20 g ; 10,25 mmoles) est obtenu sous la forme d'un solide de couleur blanche.
Rendement : 70%
Caractéristiques spectrales :
¹H RMN (DMSO) δ (ppm) : 1,40 (6H, s) ; 1,40 (3H, s) ; 2,45 (1H, d) ; 2,80 (1H, d) ; 3,33 (3H, s) ; 3,75 à 3,81 (3H, m) ; 3,85 (1H, s, échangeable par D₂O) ; 4,00 (1H, d) ; 4,22 (1H, d) ; 4,46 (2H, s) ; 5,35 (1H, s élargi); 10,90 (1H, s, échangeable par D₂O).

| Analyse élémentaire : (Formule brute : C₁₆H₂₄ClNO₉ M=409,82) | | | | |
|---|---|---|---|---|
| | **C** | **H** | **N** | **Cl** |
| **% trouvé** | 46,89 | 5,78 | 3,29 | 8,64 |
| **% calculé** | 46,89 | 5,90 | 3,42 | 8,65 |

### Exemple 5: 1,2-O-Isopropylidène-3-isopropényl-4-O-méthyl-5-O-méthylcarbamoyl-β-_{D}-psicopyranose

A une solution de 2,00 g (7,29 mmoles) du composé obtenu à l'Exemple 2 dans 40 ml de dichlorométhane anhydre refroidie à 0°C contenant 1,34 g (10,97 mmoles) de 4-diméthylaminopyridine sont ajoutés, goutte-à-goutte et sous atmosphère d'azote, 0,90 ml (0,83 g ; 14,56 mmoles) d'isocyanate de méthyle. Après 1 heure d'agitation à 0°C le mélange réactionnel est versé dans 20 ml d'eau glacée et l'ensemble est laissé sous agitation pendant 1 heure. Après traitement habituel de la phase organique, puis purification du produit brut par chromatographie sur gel de silice (éluant: éther diéthylique), 2,3 g (6,94 mmoles) du composé attendu sous la forme d'une mousse de couleur blanche sont obtenus.
Rendement : 95%
Caractéristiques spectrales :
¹H RMN (DMSO) δ (ppm) : 1,30 (3H, s) ; 1,40 (3H, s) ; 1,80 (3H, s) ; 2,60 (3H, d) ; 3,25 (3H, s) ; 3,70 à 3,75 (3H, t) ; 3,89 (1H, s, échangeable par D₂O) ; 3,90 (1H, d) ; 4,12 (1H, d) ; 5,02 (1H, s) ; 5,20 (1H, s); 5,25 (1H, s) 7,30 (1H, q, échangeable par D₂O).

### Exemple 6: 1,2-O-Isopropylidène-3-(2-méthyloxiran-2-yl)-4-O-méthyl-5-O-méthylcarbamoyl-β-_{D}-psicopyranose

Ce composé est obtenu de manière identique à celle décrite dans l'Exemple 4 à partir de 0,20 g (0,60 mmoles) du composé obtenu à l'exemple précédent, de 0,15 g (1,78 mmoles) de bicarbonate de sodium solide et 0,22 g (0.93 mmoles) d'acide 3-chloroperbenzoïque à 72%.
Rendement : 72%
Caractéristiques spectrales :
¹H RMN (DMSO) δ (ppm) : 1,35 (9H, s) ; 2,40 (1H, d) ; 2,60 (3H, s) ; 2,75 (1H, d) ; 3,30 (3H, s) ; 3,65 (1H, s, échangeable par D₂O) ; 3,70 à 3,80 (3H, m) ; 3,90 (1H, d) ; 4,20 (1H, d) ; 5,20 (1H, s élargi) ; 7,20 (1H, q, échangeable par D₂O).

| Analyse élémentaire : (Formule brute : C₁₅H₂₅NO₈ M=347,37) | | | |
|---|---|---|---|
| | **C** | **H** | **N** |
| **% trouvé** | 51,95 | 7,25 | 4,09 |
| **% calculé** | 51,87 | 7,25 | 4,03 |

### Exemple 7: 5-O-Benzoylcarbamoyl-1,2-O-isopropylidène-3-isopropényl-4-O-méthyl-β-_{D}-psicopyranose

En procédant comme décrit pour la préparation de l'Exemple 5 à partir de 1,20 g (4,37 mmol) du composé obtenu à l'exemple 2 et 0,96 g (6,52 mmoles) d'isocyanate de benzoyle, et après purification par chromatographie sur gel de silice (éluant: éther diéthylique/dichlorométhane, 2:1), 1,30 g (3,08 mmoles) de produit attendu sont obtenus.
Rendement : 70%
Caractéristiques spectrales :
¹H RMN (DMSO ) δ (ppm) : 1,30 (3H, s) ; 1,40 (3H, s) ; 1,85 (3H, s) ; 3,31 (3H, s) ; 3,72 (1H, d) ; 3,80 (1H, d) 3,89 (1H, d) ; 4,05 (1H, d) ; 4,15 (1H, d) ; 4,40 (1H, s, échangeable par D₂O) ; 5,05 (1H, s élargi); 5,25 (1H, s élargi) ;5,50 (1H, s élargi) ;7,52 (2H, t) ; 7,62 (1H, t) ; 7,90 (2H, d) ; 11,12 (1H, s, échangeable par D₂O).

### Exemple 8: 5-O-Benzoylcarbamoyl-1,2-O-isopropylidène-3-(2-méthyloxiran-2-yl)-4-O-méthyl-β-_{D}-psicopyranose

En procédant selon le mode opératoire décrit pour la préparation de l'Exemple 4, à partir de 0,25 g (0,59 mmoles) du composé obtenu à l'exemple 7 et de 0,22 g (0,93 mmoles) d'acide 3-chloroperbenzoïque à 72%, puis purification par chromatographie sur gel de silice (éluant: éther diéthylique/heptane, 5:1), 0,10 g (0,23 mmoles) de composé attendu sous la forme d'une mousse de couleur blanche sont recueillis.
Rendement : 39%
Caractéristiques spectrales :
¹H RMN (DMSO) δ (ppm) : 1,40 (9H, s) ; 2,41 (1H, d) ;2,81 (1H, d) ; 3,35 (3H, s) ; 3,70 à 3,82 (3H, m) ; 4,00 (1H, d) ; 4,25 (1H, d) ; 4,30 (1H, s, échangeable par D₂O) ; 5,40 (1H, s élargi) ; 7,52 (2H, t) ; 7,61 (1H, t) ; 7,88 (2H, d) ; 10,96 (1H, s, échangeable par D₂O).

| Analyse élémentaire : (Formule brute : C₂₁H₂₇NO₉ M=437,45) | | | |
|---|---|---|---|
| | **C** | **H** | **N** |
| **% trouvé** | 57,84 | 6,27 | 3,04 |
| **% calculé** | 57,66 | 6,22 | 3,20 |

### Exemple 9: 3-Isopropényl-4-O-méthyl-5-O-méthylcarbamoyl-β-_{D}-psicopyranose

A une suspension de 1,10 g (3,24 mmoles) du composé obtenu à l'Exemple 5 dans 40 ml d'eau sont ajoutés 20 g de résine acide (DOWEX 50X8-100). L'ensemble est chauffé à 60°C pendant 80 minutes puis laissé sous agitation à température ambiante pendant 1,5 heures. Le mélange réactionnel est filtré et le filtrat évaporé pour donner 0,92 g (3,15 mmoles) du triol attendu sous la forme d'une mousse blanche.
Rendement : 97%
Caractéristiques spectrales :
¹H RMN (DMSO) δ (ppm) : 1,80 (3H, s) ; 2,60 (3H, d) ; 3,20 (3H, s) ; 3,35 à 3,60 (4H, m, dont 1H échangeable par D₂O) ; 3,80 (1H, d) ; 4,00 (1H, m) ; 4,60 (1H, m, échangeable par D₂O) ; 4,89 (1H, s élargi) ; 5,15 (1H, s élargi) ; 5,22 (1H, s élargi) ; 5,50 (1H, s, échangeable par D₂O) ; 7,25 (1H, q, échangeable par D₂O).

### Exemple 10: 1-(para-Toluènesulfonyl)-3-isopropényl-4-O-méthyl-5-O-méthylcarbamoyl-β-_{D}-psicopyranose

A une solution de 3,90 g (13,39 mmoles) de triol obtenu à l'Exemple 9 dans 40 ml de pyridine anhydre sont ajoutés, à température ambiante et sous atmosphère d'azote, 8,94 g (46,89 mmoles) de chlorure de tosyle. Après 2 heures d'agitation à température ambiante le mélange réactionnel est versé dans 30 ml d'une solution aqueuse (10%) de chlorure d'ammonium refroidie à 0°C. Le mélange réactionnel est ensuite dilué dans 200 ml d'acétate d'éthyle puis les 2 phases sont séparées. Après traitement habituel de la phase organique et purification du produit brut par chromatographie sur gel de silice (éluant : éther diéthylique), 4,98 g (11,18 mmoles) de produit attendu sous la forme d'une mousse blanche sont obtenus.
Rendement : 83%
Caractéristiques spectrales :
¹H RMN (DMSO ) δ (ppm) : 1,70 (3H, s) ; 2,40 (3H, s) ; 2,70 (3H, d) ; 3,25 (3H, s) ; 3,60 (1H, d) ; 3,75 (1H, s échangeable par D₂O) ; 3,80 (1H, d) ; 3,90 à 4,00 (3H, m) ; 4,85 (1H, s élargi); 5,05 (1H, s élargi) ; 5,20 (1H, s élargi) ; 6,60 (1H, s, échangeable par D₂O) ; 7,22 (1H, q, échangeable par D₂O) ; 7,48 (2H, d) ; 7,75 (2H, d).

### Exemple 11: 1-(para-Toluènesulfonyl)-3-(2-méthyloxiran-2-yl)-4-O-méthyl-5-O-méthylcarbamoyl-β-_{D}-psicopyranose

En procédant selon le mode opératoire décrit pour la préparation de l'Exemple 4, à partir de 0,25 g (0,56 mmoles) du composé obtenu à l'exemple précédent et de 0,21 g (0,89 mmoles) d'acide 3-chloroperbenzoïque à 72%, puis purification par chromatographie sur gel de silice (éluant : pentane/acétate d'éthyle, 1:1), 0,16 g de produit attendu sous la forme d'une mousse de couleur blanche sont obtenus.
Rendement : 61%
Caractéristiques spectrales :
¹H RMN (DMSO) δ (ppm) : 1,80 (3H, s) ; 2,20 (1H, d) ; 2,40 (3H, s) ; 2,50 (1H, m) ; 2,55 (3H, d) ; 3,25 (3H, s) ; 3,44 (1H, s échangeable par D₂O) ; 3,60 (1H, d) ; 3,80 à 3,85 (2H, m) ; 4,00 (1H, d) ; 4,10 (1H, d) ; 5,11 (1H, s élargi) ; 6,72 (1H, s, échangeable par D₂O) ; 7,05 (1H, q, échangeable par D₂O) ; 7,49 (2H, d) ; 7,80 (2H, d).

| Analyse élémentaire : (Formule brute : C₁₉H₂₇NO₁₀S M=461,48) | | | | |
|---|---|---|---|---|
| | **C** | **H** | **N** | **S** |
| **% trouvé** | 49,83 | 6,34 | 3,15 | 6,64 |
| **% calculé** | 49,45 | 5,90 | 3,04 | 6,95 |

### Exemple 12: 1-Bromo-1-désoxy-3-isopropényl-4-O-méthyl-5-O-méthylcarbamoyl-β-_{D}-psicopyranose

A une solution de 0,31 g (0,69 mmoles) du composé obtenu à l'Exemple 10, dans 15 ml d'acétone sont ajoutés, à température ambiante et en une seule portion, 0,29 g (3,34 mmoles) de bromure de lithium. Le mélange réactionnel est placé sous agitation puis chauffé à 75°C pendant 1,5 heures. 0,26 g (2,99 mmoles) de bromure de lithium supplémentaires sont alors ajoutés et l'ensemble est à nouveau chauffé à 75°C pendant 1 heure. L'acétone est évaporée sous pression réduite et le résidu repris dans 20 ml d'acétate d'éthyle. Le traitement habituel de la phase organique fournit 0,23 g (0,65 mmoles) de produit attendu sous la forme d'une mousse de couleur blanche.
Rendement : 94%
Caractéristiques spectrales :
¹H RMN (DMSO ) δ (ppm) : 1,80 (3H, s) ; 2,60 (3H, d) ; 3,22 (3H, s) ; 3,52 (1H, d) ; 3,70 (1H, d) ; 3,78 (1H, d) ; 3,82 (1H,s échangeable par D₂O) ; 3,89 (1H, d) ; 4,05 (1H, d) ; 4,95 (1H, sélargi) ; 5,20 (1H, s élargi) ; 5,23 (1H, s élargi) ; 6,20 (1H, s, échangeable par D₂O) ; 7,30 (1H, q, échangeable par D₂O).

### Exemple 13: 1-Bromo-1-désoxy-3-(2-méthyloxiran-2-yl)-4-O-méthyl-5-O-méthylcarbamoyl-β-_{D}-psicopyranose

En procédant selon le mode opératoire décrit pour la préparation de l'Exemple 4, à partir de 0,23 g (0,65 mmoles) du composé obtenu à l'exemple précédent et de 0,23 g (0,97 mmoles) d'acide 3-chloroperbenzoïque à 72%, puis purification par chromatographie sur gel de silice (éluant : éther diéthylique), 0,16 g (0,43 mmoles) de composé attendu sous la forme d'un solide de couleur blanche sont obtenus.
Rendement : 66%
Caractéristiques spectrales :
¹H RMN (DMSO ) δ (ppm) : 1,35 (3H, s) ; 2,32 (1H, d) ; 2,58 (3H, d) ; 2,72 (1H, d) ; 3,28 (3H, s) ; 3,45 (1H,s échangeable par D₂O) ; 3,62 (1H, d) ; 3,69 (2H, s) ; 3,90 (1H, d) ; 3,99 (1H, d) ; 5,12 (1H, s élargi) ; 6,32 (1H, s, échangeable par D₂O) ; 7,10 (1H, q, échangeable par D₂O).

| Analyse élémentaire : (Formule brute : C₁₂H₂₀BrNO₇ M=370,20) | | | | |
|---|---|---|---|---|
| | **C** | **H** | **N** | **Br** |
| **% trouvé** | 38,72 | 5,18 | 3,83 | 21,64 |
| **% calculé** | 38,93 | 5,45 | 3,78 | 21,58 |

### Exemple 14: 1,2-O-Isopropylidène-3-isopropényl-4,5-di-O-méthyl-β-_{D}-psicopyranose

A une suspension d'hydrure de sodium (60% dans l'huile ; 1,10 g ; 27,50 mmoles) dans 50 ml de N,N-diméthylformamide anhydre sont ajoutés, goutte-à-goutte et à température ambiante, 3,28 g (12,60 mmoles) du composé obtenu à l'Exemple 1, en solution dans 35 ml de N,N-diméthylformamide anhydre. Le mélange réactionnel est placé sous agitation à température ambiante pendant 30 minutes puis est refroidi à 0°C avant l'addition goutte-à-goutte de 2,35 ml (5,36 g ; 37,75 mmoles) d'iodure de méthyle. Après 50 minutes d'agitation à 0°C le mélange réactionnel est versé dans 100 ml d'une solution (10%) aqueuse de chlorure d'ammonium refroidie à 0°C. Le traitement habituel de la phase organique conduit, après purification par chromatographie sur gel de silice (éluant : pentane/acétate d'éthyle, 8:1 puis 5:1), à 2,93 g (10,16 mmoles) de produit attendu sous la forme d'une huile de couleur jaune.
Rendement : 80%
Caractéristiques spectrales :
¹H RMN (DMSO ) δ (ppm) : 1,29 (3H, s) ; 1,35 (3H, s) ; 1,80 (3H, s) ; 3,30 (3H, s) ; 3,40 (3H, s) ; 3,65 (1H, d) ; 3,72 (2H, m) ; 3,95 (1H, s) ; 3,97 (1H, d) ; 4,05 (1H, d) ; 4,49 (1H, s, échangeable par D₂O) ; 5,02 (1H, s élargi) ; 5,15 (1H,s élargi).

### Exemple 15: 1,2-O-Isopropylidène-3-(2-méthyloxiran-2-yl)-4,5-di-O-méthyl-β-_{D}-psicopyranose

En procédant selon le mode opératoire décrit pour la préparation de l'Exemple 4, à partir de 0,44 g (1,52 mmoles) du composé obtenu à l'exemple 14 et de 0,40 g (1,69 mmoles) d'acide 3-chloroperbenzoïque à 72%, puis purification par chromatographie sur gel de silice (éluant : pentane/acétate d'éthyle, 1,5:1), 0,27 g (0,89 mmoles) de composé attendu sous la forme d'un solide de couleur blanche sont obtenus.
Rendement : 58%
Caractéristiques spectrales :
¹H RMN (DMSO) δ (ppm) : 1,40 (3H, s) ; 1,44 (6H, s); 2,22 (1H, d) ; 2,95 (1H, d) ; 3,35 (3H, s) ; 3,40 (3H, s) ; 3,60 (1H, d) ; 3,66 à 3,72 (2H, m) ; 3,92 (1H, d) ; 3,96 (1H, d) ; 4,40 (1H,s, échangeable par D₂O); 4,56 (1H, d).

| Analyse élémentaire : (Formule brute : C₁₄H₂₄O₇ M=304,34) | | |
|---|---|---|
| | **C** | **H** |
| **% trouvé** | 55,92 | 7,99 |
| **% calculé** | 55,25 | 7,95 |

### Exemple 16: 1,2-O-Isopropylidène-3-(2-méthyloxitan-2-yl)-4-O-méthyl-β-_{D}-psicopyranose

En procédant selon le mode opératoire décrit pour la préparation de l'Exemple 4, à partir de 0,25 g (0,91 mmoles) du composé obtenu à l'exemple 2 et de 0,34 g (1,44 mmoles) d'acide 3-chloroperbenzoïque à 72%, puis purification par chromatographie sur gel de silice (éluant : pentane/acétate d'éthyle, 2:1), 0,17 g (0,58 mmoles) de composé attendu sous la forme d'une mousse de couleur blanche sont obtenus.
Rendement : 63%
Caractéristiques spectrales :
¹H RMN (DMSO) δ (ppm) : 1,35 (9H, s) ; 2,42 (1H, d) ; 2,75 (1H, d) ; 3,20 (3H, s) ; 3,50 (1H, s) ; 3,65 à 3,85 (3H, m) ; 4,10 (1H, s élargi) ; 4,20 (1H, d) ; 4,95 (1H, s, échangeable par D₂O) ; 5,15 (1H, d, échangeable par D₂O).

| Analyse élémentaire : (Formule brute : C₁₃H₂₂O₇ M=290,31) | | |
|---|---|---|
| | **C** | **H** |
| **% trouvé** | 54,14 | 7,73 |
| **% calculé** | 53,78 | 7,64 |

### Exemple 17: 1,2-O-Isopropylidène-3-(2-méthyloxiran-2-yl)-4-O-méthyl-2,5-hexodiulo-β-_{D}-érythro-2,6-pyranose

A une solution du composé obtenu à l'Exemple 16 (0,22 g ; 0,76 mmoles) dans 5 ml de dichlorométhane anhydre contenant 0,50 g de tamis moléculaire activé (0,4 nm) sont ajoutés 0,70 g (1,86 mmoles) de dichromate de pyridinium. L'ensemble est placé sous agitation, à température ambiante pendant 22 heures puis le mélange réactionnel est directement chromatographié sur gel de silice (éluant : acétate d'éthyle/pentane, 2:1) pour donner 0,13 g (0,45 mmoles) de cétone attendue sous la forme d'un solide de couleur blanche.
Rendement : 59%
Caractéristiques spectrales :
¹H RMN (DMSO ) δ (ppm) 1,45 (9H, s) ; 2,50 (1H, m) ; 2,80 (1H, d) ; 3,50 (3H, s) ; 3,85 (1H, d) ; 3,90 (1H, d) ; 4,20 (1H, d) ; 4,30 (1H, d) ; 4,50 (1H, s) ; 4,70 (1H, s, échangeable par D₂O).

| Analyse élémentaire : (Formule brute : C₁₃H₂₀O₇ M=288,30) | | |
|---|---|---|
| | **C** | **H** |
| **% trouvé** | 53,77 | 7,05 |
| **% calculé** | 54,16 | 6,99 |

### Exemple 18: 5-O-(1-imidazolylcarbonyl)-1,2-O-isopropylidène-3-(2-méthyloxiran-2-yl)-4-O-méthyl-β-_{D}-psicopyranose

A une solution du composé obtenu à l'Exemple 16 (2,00 g ; 6,89 mmoles) dans 40 ml de dichlorométhane anhydre sont ajoutés en une seule portion, sous atmosphère d'azote et à température ambiante, 3,35 g (20,66 mmoles) de carbonyle diimidazole. Le mélange réactionnel est placé sous agitation à température ambiante pendant 5 heures. Le solvant est alors évaporé sous pression réduite et le résidu solide résultant est chromatographié sur gel de silice (éluant: acétate d'éthyle/pentane, 2:1). 2,33 g (6,06 mmoles) de composé attendu sous la forme d'une mousse blanche sont ainsi obtenus.
Rendement : 88%
Caractéristiques spectrales :
¹H RMN (DMSO ) δ (ppm) : 1,40 (9H, 2s) ; 2,80 (1H, m) ; 2,84 (1H, d) ; 3,40 (3H, s) ; 3,82 (1H, d) ; 3,90 (1H, d) ; 4,02 (2H, m) ; 4,25 (1H, d) ; 4,45 (1H, s, échangeable par D₂O) ; 5,50 (1H, s élargi) ;7,06 (1H, s) ; 7,65 (1H, s) ; 8,30 (1H, s).

| Analyse élémentaire : (Formule brute : C₁₇H₂₄N₂O₈ M=384,39) | | | |
|---|---|---|---|
| | **C** | **H** | **N** |
| **% trouvé** | 53,04 | 6,30 | 7,05 |
| **% calculé** | 53,12 | 6,29 | 7,29 |

### Exemple 19: 5-O-{1-[4-((2,3,4-Triméthoxybenzyl)pipérazinyl)]carbonyl}-1,2-O-isopropylidène-3-(2-méthyloxiran-2-yl)-4-O-méthyl-β-_{D}-psicopyranose

A une solution du composé obtenu à l'Exemple 18 (1,00 g ; 2,60 mmoles) dans 6 ml de dichlorométhane anhydre sont ajoutés, à température ambiante et sous atmosphère d'azote, 1,50 g (5,63 mmoles) de 4-[(2,3,4-triméthoxybenzyl)pipérazine] (TRIMETAZIDINE®). Le mélange réactionnel est placé sous agitation à température ambiante pendant 17 heures puis le solvant est évaporé sous pression réduite. Le résidu huileux de couleur jaune obtenu est purifié sur colonne de gel de silice (éluant: acétate d'éthyle). 1.15 g (1,97 mmoles) de produit attendu sous la forme d'une mousse blanche sont isolés.
Rendement : 75%
Caractéristiques spectrales :
¹H RMN (DMSO ) δ (ppm) : 1,40 (9H, 2s) ; 2,25 (4H, s large) ; 2,43 (1H, m) ; 2,80 (1H, d) ; 3,35 (6H, m) ; 3,60 (1H, s, échangeable par D₂O) ; 3,70 (1H, m) ; 3,71 (3H, s) ; 3,75 (2H, m) ; 3,78 (9H,s) ; 3,90 (1H,d) ; 4,19 (1H,d) ; 5,15 (1H,d) ; 6,77 (1H,d) ; 6,96 (1H,d).

| Analyse élémentaire : (Formule brute : C₂₈H₄₂N₂O₁₁ M=582,65) | | | |
|---|---|---|---|
| | **C** | **H** | **N** |
| **% trouvé** | 57,69 | 7,16 | 4,72 |
| **% calculé** | 57,72 | 7,27 | 4,81 |

### Exemple 20: 5-O-[1-(4-Chloroacétylpipérazinyl)carbonyl]-1,2-O-isopropylidène-3-(2-méthyloxiran-2-yl)-4-O-méthyl-β-_{D}-psicopyranose

A une solution du composé obtenu à l'Exemple 19 (0,50 g ; 0,86 mmoles) dans 12 ml d'un mélange acétonitrile/eau (2:1) sont ajoutés, à 0°C, 1,5 g (2,74 mmoles) de nitrate d'ammonium cérique. Le mélange réactionnel est placé sous agitation pendant 2,5 heures en laissant la température évoluer progressivement vers la température ambiante. Après évaporation des solvants le résidu est chromatographié sur gel de silice (éluant : dichlorométhane/méthanol/ammoniaque, 90:10:0,5). 0,16 g (0,40 mmoles) de composé débenzylé sont obtenus. A une solution de 0,15 g (0,37 mmoles) de ce dérivé dans 5 ml de dichlorométhane anhydre contenant 0,15 ml (0,11 g; 1,08 mmoles) de triéthylamine anhydre sont additionnés goutte-à-goutte, à 0°C et sous atmosphère d'azote, 0,045 ml (0,064 g ; 0,56 mmoles) de chlorure d'acide chloroacétique. Le mélange réactionnel est placé sous agitation à 0°C pendant 1 heure 45 minutes puis l'ensemble est versé dans une solution aqueuse de chlorure d'ammonium (10%) refroidie à 0°C. Après traitement habituel de la phase organique puis purification par chromatographie sur gel de silice (éluant : acétate d'éthyle), 0,086 g (0,18 mmoles) de produit attendu sous la forme d'une mousse beige sont obtenus.
Rendement global : 22%
Caractéristiques spectrales :
¹H RMN (DMSO ) δ (ppm) : 1,40 (9H, 3s) ; 2,43 (1H, d) ; 2,80 (1H, d) ; 3,30 (3H, s); 3,40 (8H, s élargi) ; 3,80 (4H, m, dont 1 échangeable par D₂O) ; 3,92 (1H, d) ; 4,20 (1H, d) ; 4,40 (2H, s) ; 5,18 (1H, s élargi).

| Analyse élémentaire : (Formule brute : C₂₀H₃₁ClN₂O₉ M=478,93) | | | | |
|---|---|---|---|---|
| | **C** | **H** | **N** | **Cl** |
| **% trouvé** | 49,80 | 6,41 | 5,71 | 7,73 |
| **% calculé** | 50,16 | 6,52 | 5,85 | 7,40 |

### Exemple 21: 5-O-[1-(4-Formylpipérazinyl)carbonyl]-1,2-O-isopropylidène-3-(2-méthyloxiran-2-yl)-4-O-méthyl-β-_{D}-psicopyranose

Cette synthèse a été réalisée dans les mêmes conditions opératoires que celles décrites pour la préparation de l'Exemple 19, à partir de 0,50 g du composé obtenu à l'Exemple 18 (1,30 mmoles) et de 0,40 ml (0,44 g ; 3,88 mmoles) de N-formylpipérazine dans 5 ml de dichlorométhane anhydre. 0,36 g (0,84 mmoles) de produit attendu sous la forme d'une poudre de couleur blanche sont obtenus.
Rendement : 64%
Caractéristiques spectrales :
¹H RMN (DMSO ) δ (ppm) : 1,40 (9H, s) ; 2,48 (1H, d) ; 2,80 (1H, d) ; 3,30 (3H, s) ; 3,35 (8H, m) ; 3,70 à 3,85 (4H, m, dont 1H échangeable par D₂O) ; 3,92 (1H, d) ; 4,21 (1H, d) ; 5,20 (1H, s élargi) ; 8,06 (1H, s).

| Analyse élémentaire : (Formule brute : C₁₉H₃₀N₂O₉ M=430,46) | | | |
|---|---|---|---|
| | **C** | **H** | **N** |
| **% trouvé** | 52,95 | 6,99 | 6,51 |
| **% calculé** | 53,02 | 7,02 | 6,51 |

### Exemple 22: 4,5-di-O-Chloroacétylcarbamoyl-1,2-O-isopropylidène-3-isopropényl-β-_{D}-psicopyranose

En procédant selon le mode opératoire décrit pour la préparation de l'Exemple 3, à partir de 0,50 g (1,82 mmoles) du composé obtenu à l'exemple 1 et de 0,50 ml (0,70 g ; 5,87 mmoles) d'isocyanate de chloroacétyle, puis purification par chromatographie sur gel de silice (éluant : acétate d'éthyle/pentane, 1:1), 0,90 g (1,80 mmoles) de produit attendu sous la forme d'un solide de couleur blanche sont obtenus.
Rendement : 98%
Caractéristiques spectrales :
¹H RMN (DMSO ) δ (ppm) : 1,30 (3H, s) ; 1,40 (3H,s) ; 1,75 (3H, s) ; 3,85 (2H, m) ; 4,15 (2H, m) ; 4,25 (1H, s, échangeable par D₂O) ; 4,30 (2H, s) ; 4,50 (2H, s) ; 5,15 (1H, s) ; 5,25 (2H, s); 5,30 (1H, s) ; 11,10 (1H, s, échangeable par D₂O) ; 11,18 (1H, s, échangeable par D₂O).

| Analyse élémentaire : (Formule brute : C₁₈H₂₄Cl₂N₂O₁₀ M=499,30) | | | | |
|---|---|---|---|---|
| | **C** | **H** | **N** | **Cl** |
| **% trouvé** | 43,70 | 4,84 | 5,56 | 14,24 |
| **% calculé** | 43,30 | 4,94 | 5,61 | 14,20 |

### Exemple 23: 4,5-di-O-Chloroacétylcarbamoyl-1,2-O-isopropylidène-3-(2-méthyloxiran-2-yl)-β-_{D}-psicopyranose

En procédant selon le mode opératoire décrit pour la préparation de l'Exemple 4, à partir de 0,30 g (0,60 mmoles) du produit décrit exemple 22 et de 0,17 g ( 0,72 mmoles) d'acide 3-chloroperbenzoïque à 72%, puis purification par chromatographie sur gel de silice (éluant : éther diéthylique), 0,19 g (0,38 mmoles) de produit attendu sous la forme d'une mousse de couleur blanche sont obtenus.
Rendement : 63%
Caractéristiques spectrales :
¹H RMN (DMSO) δ (ppm) : 1,35 (3H, s) ; 1,41 (6H, 2s) ; 2,50 (1H + DMSO, s élargi) ; 2,99 (1H, d) ; 3,72 (1H, d) ; 3,85 (1H, s) ; 4,00 (1H, s, échangeable par D₂O) ; 4,12 (1H, d) ; 4,25 (1H, d) ; 4,40 (2H, s) ; 4,52 (2H, s) ; 5,20 (1H, s élargi) ; 5,30 (1H, s élargi) ; 11,06 (1H, s, échangeable par D₂O) ; 11,20 (1H, s, échangeable par D₂O).

| Analyse élémentaire : (Formule brute : C₁₈H₂₄Cl₂N₂O₁₁ M=515,30) | | | | |
|---|---|---|---|---|
| | **C** | **H** | **N** | **Cl** |
| **% trouvé** | 41,78 | 4,83 | 5,26 | 14,04 |
| **% calculé** | 41,96 | 4,69 | 5,44 | 13,76 |

### Exemple 24 : 5-O-Phénylcarbamoyl-1,2-O-isopropylidène-3-(2-méthyloxiran-2-yl)-4-O-méthyl-β-_{D}-psicopyranose

Composé obtenu à partir du composé décrit à l'Exemple 2, selon le mode opératoire décrit à l'Exemple 3, en remplaçant l'isocyanate de chloroacétyle par l'isocyanate de phényle, puis en procédant à l'oxydation décrite à l'Exemple 4.

| Analyse élémentaire : (Formule brute : C₂₀H₂₇NO₈ M=409,44) | | | |
|---|---|---|---|
| | **C** | **H** | **N** |
| **% trouvé** | 58,95 | 6,39 | 3,48 |
| **% calculé** | 58,67 | 6,65 | 3,42 |

### Exemple 25: 1,2-O-Isopropylidène-3-isopropényl-3,4-di-O-méthyl-β-_{D}-psicopyranose

### Etape A : 5-O-tert-Butyldiméthysilyl-1,2-O-isopropylidène-3-isopropényl-3,4-di-O-méthyl-β-_{D}-psicopyranose

A une solution de 2,00 g (7,29 mmoles) du composé obtenu à l'Exemple 2 dans 10 ml de N,N-diméthylformamide anhydre refroidie à 0 °C sont ajoutés successivement 1,24 g (18,21 mmoles) d'imidazole et 1,92 g (12,73 mmoles) de chlorure de *tert*-butyldiméthylsilyle. L'ensemble est placé sous agitation en laissant la température évoluer vers la température ambiante pendant 72 heures. Le produit intermédiaire est isolé et purifié par chromatographié sur gel de silice (éluant : éther de pétrole/acétate d'éthyle, 4:1) pour donner 2,72 g (7,00 mmoles) de composé attendu sous la forme d'un solide de couleur blanche. Une solution de ce produit (1,18 g ; 3,04 mmoles) dans 5 ml de tétrahydrofurane anhydre est ajoutée à une suspension d'hydrure de sodium (0,22 g ; 5,50 mmoles) dans 6 ml de tétrahydrofurane anhydre. L'ensemble est placé sous agitation et atmosphère d'azote à reflux du solvant pendant 55 minutes et 1,15 ml (2,62 g ; 18,47 mmoles) d'iodure de méthyle sont ajoutés. Le mélange réactionnel est chauffé à reflux du tétrahydrofurane pendant 1,5 heures supplémentaires puis l'ensemble est versé dans une solution aqueuse de chlorure d'ammonium (10%) refroidie à 0°C. Après traitement habituel de la phase organique et purification par chromatographie sur gel de silice (éluant: dichloro-méthane), 0,86 g (2,14 mmoles) de dérivé attendu sous la forme d'une mousse de couleur blanche sont obtenus.
Rendement : 67%
Caractéristiques spectrales :
¹H RMN (DMSO ) δ (ppm) : 0,10 (6H, s) ; 0,30 (9H, s) ; 1,20 (3H, s) ; 1,30 (3H, s) ; 1,70 (3H,s ) ; 3,30 (3H, s) ; 3,35 (3H, s,) ; 3,50 à 3,60 (2H, m) ; 3,65 (1H, d) ; 3,70 (1H, d) ; 4,10 (1H, d) ; 4,20 (1H, s élargi) ; 5,00 (1H, s élargi) ; 5,20 (1H, s).

### Etape B : 1,2-O-Isopropylidène-3-isopropényl-3,4-di-O-méthyl-β-_{D}-psicopyranose

A une solution du composé obtenu à l'Etape précédente (0,85 g ; 2,11 mmoles) dans 6 ml de tétrahydrofurane anhydre refroidie à 0°C sont ajoutés rapidement 6,5 ml d'une solution (1 M dans le tétrahydrofurane) de fluorure de tétrabutylammonium (6,50 mmoles). L'ensemble est placé sous agitation en laissant évoluer la température vers la température ambiante pendant 5 heures. Après traitement habituel de la phase organique et purification par chromatographie sur gel de silice (éluant : pentane/éther diéthylique, 1:1), 0,60 g (2,09 mmoles) de composé attendu sous la forme d'une huile incolore sont obtenus.
Rendement : 99%
Caractéristiques spectrales :
¹H RMN (DMSO ) δ (ppm) : 1,30 (3H, s) ; 1,40 (3H, s) ; 1,90 (3H,s ) ; 3,30 (3H, s) ; 3,40 (3H, s,) ; 3,65 à 3,75 (3H, m) ; 3,80 (1H, dxd); 4,20 (1H, m) ; 4,30 (2H, m, dont 1 échangeable par D₂O ) ; 5,00 (1H, s élargi) ; 5,30 (1H,s élargi).

### Exemple 26: 5-O-Chloroacétylcarbamoyl-1,2-O-isopropylidène-3-isopropényl-3,4-di-O-méthyl-β-_{D}-psicopyranose

En procédant selon le mode opératoire décrit pour la préparation de l'Exemple 3, à partir de 0,10 g (0,35 mmoles) du composé obtenu à partir de l'Exemple 25 et de 0,05 ml (0,07 g ; 0,59 mmoles) d'isocyanate de chloroacétyle, une purification par chromatographie sur gel de silice (éluant : éther diéthylique), fournit 0,14 g (0,34 mmoles) de produit attendu sous la forme d'une mousse de couleur blanche.
Rendement : 97%
Caractéristiques spectrales :
¹H RMN (DMSO ) δ (ppm) : 1,30 (3H, s) ; 1,40 (3H, s) ; 1,90 (3H,s ) ; 3,30 (3H, s) ; 3,40 (3H, s,) ; 3,75 (1H, d) ; 3,80 (1H, d) ; 3,95 (1H, d) ; 4,00 (1H, d) ; 4,25 (1H, d) ; 4,45 (2H, s) ; 5,05 (1H, s) ; 5,35 (1H,s ) ; 5,40 (1H, s élargi) ; 11,10 (1H, s, échangeable par D₂O).

### Exemple 27: 1,2-O-Isopropylidène-3,4-di-O-méthyl-3-(2-méthyloxiran-2-yl)-β-_{D}-psicopyranose

En procédant selon le mode opératoire décrit pour la préparation de l'Exemple 4, à partir de 0,27 g (0,94 mmoles) du produit décrit Exemple 25 et de 0,81 g ( 3,38 mmoles) d'acide 3-chloroperbenzoïque à 72%, puis purification par chromatographie sur gel de silice (éluant : éther diéthylique), 0,25 g (0,82 mmoles) de produit attendu sous la forme d'un solide de couleur blanche sont obtenus.
Rendement : 87%
Caractéristiques spectrales :
¹H RMN (DMSO) δ (ppm) : 1,40 (9H, 2s) ; 2,45 (1H, d) ; 2,90 (1H, d) ; 3,35 (6H, 2s) ; 3,60 (1H, d) ; 3,65 (1H, dd) ; 3,85 (1H, d) ; 3,90 (1H, dd) ; 4,00 (1H, m) ; 4,40 (1H, d, échangeable par D₂O) ; 4,55 (1H, d).

### Exemple 28: 5-O-Chloroacétylcarbamoyl-1,2-O-isopropylidène-3,4-di-O-méthyl-3-(2-méthyloxiran-2-yl)-β-_{D}-psicopyranose

En procédant selon le mode opératoire décrit pour la préparation de l'Exemple 3, à partir de 0,23 g (0,75 mmoles) du produit obtenu à l'Exemple 27 et de 0,08 ml (0,11 g ; 0,94 mmoles) d'isocyanate de chloroacétyle, puis purification par chromatographie sur gel de silice (éluant : dichlorométhane/acétate d'éthyle, 4:1), 0,25 g (0,59 mmoles) de produit attendu sous la forme d'une mousse de couleur blanche sont obtenus.
Rendement : 78%
Caractéristiques spectrales :
¹H RMN (DMSO) δ (ppm) : 1,35 (9H, 2s) ; 2,50 (1H, d) ; 2,80 (1H, d) ; 3,35 (6H, 2s) ; 3,60 (1H, d) ; 3,72 (2H, 2d) ; 4,00 (1H, d) ; 4,40 (2H, s) ; 4,55 (1H, d) ; 5,35 (1H, s élargi) ; 11,02 (1H, s, échangeable par D₂O).

| Analyse élémentaire : (Formule brute : C₁₇H₂₆ClNO₉ M=423,85) | | | | |
|---|---|---|---|---|
| | **C** | **H** | **N** | **Cl** |
| **% trouvé** | 49,84 | 6,41 | 3,23 | 8,26 |
| **% calculé** | 48,17 | 6,18 | 3,30 | 8,37 |

### Exemple 29: 3-Isopropényl-4,5-di-O-méthyl-β-_{D}-psicopyranose

En procédant selon le mode opératoire décrit pour la préparation de l'Exemple 9, à partir de 1,00 g (3,47 mmoles) du composé obtenu à l'Exemple 14 et de 25 g de résine acide (DOWEX 50X8-100) chauffés dans 50 ml d'eau, puis purification par chromatographie sur gel de silice (éluant : éther diéthylique puis acétate d'éthyle), 0,59 g (2,38 mmoles) de produit attendu sous la forme d'un solide de couleur blanche sont obtenus.
Rendement : 68%
Caractéristiques spectrales :
¹H RMN (DMSO ) δ (ppm) ; 1,75 (3H, s) ; 3,30 (3H, s) ; 3,35 (1H, m) ; 3,52 (3H, s) ; 3,55 (1H, dd) ; 3,70 à 3,90 (4H, m) ; 4,30 (1H, s, échangeable par D₂O) ; 4,50 (1H, t, échangeable par D₂O) ; 4,86 (1H, s) ; 5,10 (1H, s) ; 5,40 (1H, s, échangeable par D₂O).

### Exemple 30: 1-para-Toluènesulfonyl-3-isopropényl-4,5-di-O-méthyl-β-_{D}-psicopyranose

En procédant selon le mode opératoire décrit pour la préparation de l'Exemple 10, à partir de 0,80 g (3,22 mmoles) du composé obtenu à l'exemple 29 et de 1,53 g de chlorure de tosyle dans 15 ml de pyridine anhydre, puis purification par chromatographie sur gel de silice (éluant: éther diéthylique/pentane, 2:1), 0,92 g (2,28 mmoles) de produit attendu sous la forme d'une mousse de couleur blanche sont obtenus.
Rendement : 70%
Caractéristiques spectrales :
¹H RMN (DMSO) δ (ppm) ; 1,62 (3H, s) ; 2,40 (3H, s) ; 3,22 (3H, s) ; 3,34 (3H, s) ; 3,68 (1H, d) ; 3,75-3,85 (3H, m) ; 3,90 (1H, d) ; 3,98 (1H, d) ; 4,40 (1H, s, échangeable par D₂O) ; 4,81 (1H, s élargi) ; 4,95 (1H, d) ; 6,46 (1H, s, échangeable par D₂O) ; 7,45 (2H, d) ; 7,71 (2H, d).

### Exemple 31: 1-Désoxy-1-iodo-3-isopropényl-4,5-di-O-méthyl-β-_{D}-psiopyranose

En procédant selon le mode opératoire décrit pour la préparation de l'Exemple 12, à partir de 0,10 g (0,25 mmoles) du composé obtenu à l'exemple 30 et de 0,18 g (1,20 mmol) d'iodure de sodium dans 3 ml d'acétone à température ambiante, puis purification par chromatographie sur gel de silice (éluant : éther diéthylique/pentane, 1,5:1), 0,079 g (0,22 mmoles) de produit attendu sous la forme d'un solide de couleur jaune pâle sont obtenus.
Rendement : 88%
Caractéristiques spectrales :
¹H RMN (DMSO) δ (ppm) : 1,76 (3H, s) ; 3,25 (4H, s + d) ; 3,40 (3H, s) ; 3,64 (1H, d) ; 3,75 à 3,95 (4H, m) ; 4,50 (1H, s, échangeable par D₂O) ; 4,95 (1H, s élargi) ; 5,15 (1H, s élargi) ; 5,98 (1H, s, échangeable par D₂O).

### Exemple 32: (1S,6R,7R,8R)-8-Isopropényl-6,7-diméthoxy-1,4-dioxaspiro[2,5]octan-8-ol

A une solution du composé obtenu à l'Exemple 31 (0,52 g ; 1,45 mmol) dans 5 ml de dioxane sont ajoutés, par portions et en 48 heures, 2,20 g (9,49 mmol) d'oxyde d'argent (I). Le mélange réactionnel est alors filtré et le filtrat évaporé pour donner 0,32 g (1,39 mmol) de produit attendu sous la forme d'un solide blanc.
Rendement : 93%
Caractéristiques spectrales :
¹H RMN (DMSO ) δ (ppm) : 1,72 (3H, s) ; 2,71 (2H, s) ; 3,40 (6H, 2s) ; 3,70 (1H, dd) ; 3,79 à 3,90 (2H, m) ; 3,95 (1H, dd) ; 4,70 (1H, s, échangeable par D₂O) ; 5,00 (1H, s élargi) ; 5,20 (1H, s élargi).

| Analyse élémentaire : (Formule brute : C₁₁H₁₈O₅ M=230,26) | | |
|---|---|---|
| | **C** | **H** |
| **% trouvé** | 57,20 | 7,89 |
| **% calculé** | 57,38 | 7,88 |

### Exemple 33: 5-O-Chloroéthylcarbamoyl-1,2-O-isopropylidène-3-isopropényl-4-O-méthyl-β-_{D}-psicopyranose

En procédant selon le mode opératoire décrit pour la préparation de l'Exemple 3, à partir de 1,00 g (3,64 mmoles) du composé obtenu à l'Exemple 2 et de 0,46 ml (0,57 g ; 5,40 mmoles) d'isocyanate de chloroéthyle, 1,35 g (3,55 mmoles) de produit attendu sous la forme d'un solide de couleur blanche sont obtenus.
Rendement : 97%
Caractéristiques spectrales :
¹H RMN (DMSO) δ (ppm) : 1,30 (3H, s) ; 1,35 (3H,s); 1,80 (3H, s) ; 3,25 (3H, s) ; 3,32 (2H, m) ; 3,61 (2H, m) ; 3,72 (1H, d) ; 3,78 (2H, m) ; 3,90 (1H, s, échangeable par D₂O) ; 3,95 (1H, d) ; 4,12 (1H, d) ; 5,08 (1H, s) ; 5,25 (1H, s élargi) ; 5,28 (1H, s élargi) ; 7,75 (1H, t, échangeable par D₂O).

### Exemple 34: 5-O-Chloroéthylcarbamoyl-1,2-O-isopropylidène-3-(2-méthyloxiran-2-yl)-4-O-méthyl-β-_{D}-psicopyranose

En procédant selon le mode opératoire décrit pour la préparation de l'Exemple 4, à partir de 0,25 g (0,66 mmoles) du composé obtenu à l'Exemple 33 et de 0,17 g (0,71 mmoles) d'acide 3-chloroperbenzoïque à 72%, puis purification par chromatographie sur gel de silice (éluant : éther diéthylique), 0,16 g (0,40 mmoles) de composé attendu sous la forme d'une mousse de couleur blanche sont obtenus.
Rendement : 60%
Caractéristiques spectrales :
¹H RMN (DMSO) δ (ppm) : 1,40 (9H, s) ; 2,42 (1H, d) ; 2,81 (1H, d) ; 3,30 (5H, s) ; 3,60 à 3,75 (4H, m, dont 1H échangeable par D₂O) ; 3,80 (1H, d) ; 3,90 (1H, d) ; 4,20 (1H, d) ; 5,20 (1H, s élargi) ; 7,60 (1H, t, échangeable par D₂O).

| Analyse élémentaire : (Formule brute : C₁₆H₂₆ClNO₈ M=395,84) | | | | |
|---|---|---|---|---|
| | **C** | **H** | **N** | **Cl** |
| **% trouvé** | 48,34 | 6,43 | 3,68 | 9,27 |
| **% calculé** | 48,55 | 6,62 | 3,54 | 8,96 |

### Exemple 35: 5-O-(2,4-Difluorophénylcarbamoyl)-1,2-O-isopropylidène-3-isopropényl-4-O-méthyl-β-_{D}-psicopyranose

En procédant selon le mode opératoire décrit pour la préparation de l'Exemple 3, à partir de 0,50 g (1,82 mmoles) du composé obtenu à l'Exemple 2 et de 0,43 ml (0,56 g ; 3,63 mmoles) d'isocyanate de 2,4-difluorophényle, puis purification par chromatographie sur gel de silice (éluant : pentane/éther diéthylique, 4:1), 0,75 g (1,75 mmoles) de produit attendu sous la forme d'un solide de couleur blanche sont obtenus.
Rendement : 96%
Caractéristiques spectrales :
¹H RMN (DMSO ) δ (ppm) : 1,30 (3H, s) ; 1,40 (3H,s); 1,85 (3H, s) ; 3,30 (3H, s) ; 3,80 (2H, s); 3,82 (1H, d) ; 4,00 (1H, d) ; 4,10 (1H, s, échangeable par D₂O) ; 4,15 (1H, d) ; 5,08 (1H, s élargi) ; 5,30 (1H, s élargi) ; 5,40 (1H, s élargi) ;7,05 (1H, txd) ; 7,31 (1H, txd) ; 7,81 (1H, m) ; 9,70 (1H, s, échangeable par D₂O).

### Exemple 36: 5-O-(2,4-Difluorophénylcarbamoyl)-1,2-O-isopropylidène-3-(2-méthyloxiran-2-yl)-4-O-méthyl-β-_{D}-psicopyranose

En procédant selon le mode opératoire décrit pour la préparation de l'Exemple 4, à partir de 0,72 g (1,67 mmoles) du composé obtenu à l'Exemple 35 et de 0,45 g (1,88 mmoles) d'acide 3-chloroperbenzoïque à 72%, puis purification par chromatographie sur gel de silice (éluant: éther diéthylique/éther de pétrole, 1:1), 0,32 g (0,72 mmoles) de composé attendu sous la forme d'une mousse de couleur blanche sont obtenus.
Rendement : 43%
Caractéristiques spectrales :
¹H RMN (DMSO ) δ (ppm) : 1,40 (9H, s) ; 2,45 (1H, d) ; 2,82 (1H, d) ; 3,36 (3H, s) ; 3,80 (1H, d) ; 3,82 (2H, s); 3,95 (1H, s, échangeable par D₂O) ; 4,00 (1H, d) ; 4,25 (1H, d) ; 5,30 (1H, s élargi) ; 7,05 (1H,txd) ; 7,30 (1H, txd) ; 7,80 (1H, m) ; 9,50 (1H, s, échangeable par D₂O).

| Analyse élémentaire : (Formule brute : C₂₀H₂₅F₂NO₈ M=445,42) | | | |
|---|---|---|---|
| | **C** | **H** | **N** |
| **% trouvé** | 53,65 | 5,42 | 3,16 |
| **% calculé** | 53,93 | 5,66 | 3,14 |

### Exemple 37: 5-O-(3-Trifluorométhylphénylcarbamoyl)-1,2-O-isopropylidène-3-(2-méthyloxiran-2-yl)-4-O-méthyl-β-_{D}-psicopyranose

En procédant selon le mode opératoire décrit pour la préparation de l'Exemple 3, à partir de 0,25 g (0,86 mmoles) du composé obtenu à l'Exemple 2 et de 0,25 ml (0,34 g; 1,82 mmoles) d'isocyanate de 3-trifluorométhylphényle, puis oxydation selon le mode opératoire décrit à l'exemple 4, et enfin purification par chromatographie sur gel de silice (éluant : pentane/acétate d'éthyle, 4:1), 0,23 g (0,50 mmoles) de composé attendu sous la forme d'une mousse de couleur blanche sont obtenus.
Rendement global : 58%
Caractéristiques spectrales :
¹H RMN (DMSO) δ (ppm) : 1,40 (9H, s) ; 2,45 (1H, d) ; 2,82 (1H, d) ; 3,40 (3H, s) ; 3,60 (1H, s, échangeable par D₂O) ; 3,80 (3H, d + s) ; 4,00 (1H, d) ; 4,25 (1H, d) ; 5,46 (1H, s élargi) ; 7,32 (1H, d) ; 7,53 (1H, t) ; 7,65 (1H, d) ; 7,92 (1H, s) ; 10,03 (1H, s, échangeable par D₂O).

| Analyse élémentaire : (Formule brute : C₂₁H₂₆F₃NO₈ M=477,43) | | | |
|---|---|---|---|
| | **C** | **H** | **N** |
| **% trouvé** | 53,33 | 5,34 | 3,17 |
| **% calculé** | 52,83 | 5,49 | 2,93 |

### Exemple 38: 5-O-(1-Naphtylcarbamoyl)-1,2,O-isopropylidène-3-(2-méthyloxiran-2-yl)-4-O-méthyl-β-_{D}-psicopyranose

En procédant selon le mode opératoire décrit pour la préparation de l'Exemple 3, à partir de 0,25 g (0,86 mmoles) du composé obtenu à l'Exemple 2 et de 0,26 ml (0,30 g; 1,79 mmoles) d'isocyanate de 1-naphtyle, puis oxydation selon le mode opératoire décrit à l'exemple 4, et enfin purification par chromatographie sur gel de silice (éluant : dichlorométhane/acétate d'éthyle, 4:1), 0,18 g (0,39 mmoles) de composé attendu sous la forme d'un solide de couleur blanche sont obtenus.
Rendement global : 45%
Caractéristiques spectrales :
¹H RMN (DMSO ) δ (ppm) : 1,42 (9H, s) ; 2,49 (1H, d) ; 2,85 (1H, d) ; 3,40 (3H, s) ; 3,85 (4H, m, dont 1H échangeable par D₂O) ; 4,00 (1H, d) ; 4,26 (1H, d) ; 5,36 (1H, s élargi) ; 7,45 à 7,60 (3H, m) ; 7,72 (2H, q) ; 7,95 (1H, m) ; 8,15 (1H, m) ; 9,62 (1H, s, échangeable par D₂O).

| Analyse élémentaire : (Formule brute : C₂₄H₂₉NO₈ M=459,50) | | | |
|---|---|---|---|
| | **C** | **H** | **N** |
| **% trouvé** | 62,23 | 6,30 | 3,20 |
| **% calculé** | 62,73 | 6,36 | 3,05 |

### Exemple 39: 1,2-O-isopropylidène-3-O-allyl-β-_{D}-fructopyranose

### Etape A : 1,2:4,5-di-O-isopropylidène-3-O-allyl-β-_{D}-fructopyranosee

A une suspension d'hydrure de sodium (60% dans l'huile ; 2,30 g ; 57,50 mmoles) dans 60 ml de N,N-diméthylformamide anhydre sont ajoutés, goutte-à-goutte et à température ambiante, 10,00 g (38,42 mmoles) de 1,2:4,5-di-O-isopropylidène-β-_{D}-fructopyranose, en solution dans 20 ml de N,N-diméthylformamide anhydre. Le mélange réactionnel est placé sous agitation à température ambiante pendant 2 heures et on y additionne goutte-à-goutte 6,70 ml (9,37 g ; 77,45 mmoles) de bromure d'allyle. Après 75 minutes d'agitation à température ambiante le mélange réactionnel est versé dans 100 ml d'une solution (10%) aqueuse de chlorure d'ammonium refroidie à 0°C. Le traitement habituel de la phase organique, puis une purification par chromatographie sur gel de silice (éluant : pentane/éther diéthylique, 8:1) fournit 9,88 g (32,89 mmoles) de produit attendu sous la forme d'une huile de couleur jaune.
Rendement : 85%

### Etape B : 1,2-O-isopropylidène-3-O-allyl-β-_{D}-fructopyranose

En procédant selon le mode opératoire décrit pour la préparation de l'Exemple 1, Etape C, à partir de 9,30 g (30,96 mmoles) du composé obtenu à l'Etape précédente, puis purification par chromatographie sur gel de silice (éluant acétate d'éthyle/éther diéthylique, 2:1), 6,04 g (23,20 mmoles) de produit attendu sous la forme d'un solide de couleur blanche sont obtenus.
Rendement : 75%
Caractéristiques spectrales :
¹H RMN (DMSO ) δ (ppm) : 1,30 (3H, s) ; 1,35 (3H, s) ; 3,40 (1H, d) ; 3,50 (1H, d) ; 3,65 (2H, m) ; 3,70 (2H, d) ; 3,90 (1H, d) ; 4,03 (1H, dxd) ; 4,35 (1H, dxd) ; 4,70 (1H, d, échangeable par D₂O) ; 4,82 (1H, d, échangeable par D₂O) ; 5,10 (1H, d) ; 5,25 (1H, d) ; 5,90 (1H, txdxd).

### Exemple 40: 1,2-O-Isopropylidène-3-O-allyl-4-O-méthyl-β-_{D}-fructopyranose

En procédant selon le mode opératoire décrit pour la préparation de l'Exemple 2, à partir de 1,00 g (3,84 mmoles) du composé obtenu à l'Exemple 39 et de 1,91 g de dibutylétain (7,67 mmoles) à reflux pendant 48 heures dans 30 ml de méthanol puis, en utilisant l'intermédiaire formé à l'issue de cette réaction et en le soumettant à l'action de 1,20 ml (2,74 g ; 19,27 mmoles) d'iodure de méthyle à reflux du dioxane (20 ml) pendant 60 heures, 0,92 g (3,35 mmoles) de composé attendu sous la forme d'une mousse de couleur blanche sont isolés après chromatographie sur gel de silice (éluant : pentane/acétate d'éthyle, 2:1).
Rendement : 87%
Caractéristiques spectrales :
¹H RMN (DMSO) δ (ppm) : 1,28 (3H, s) ; 1,38 (3H, s) ; 3,30 (3H, s) ; 3,32 (1H, dxd) ; 3,52 (2H, d+s) ; 3,70 (1H, d) ; 3,80 (1H, d) ; 3,92 (2H, m) ; 4,00 (1H, d) ; 4,28 (1H, dxd) ; 4,73 (1H, d, échangeable par D₂O) ; 5,10 (1H, d) ; 5,21 (1H, d) ; 5,90 (1H, txdxd).

### Exemple 41: 1,2-O-Isopropylidène-3-[(2SR),(2:3-époxypropyl)]-4-O-méthyl-β-_{D}-fructopyranose

En procédant selon le mode opératoire décrit pour la préparation de l'Exemple 4, à partir de 1,18 g (4,30 mmoles) du composé obtenu à l'Exemple 40 et de 1,90 g (8,03 mmoles) d'acide 3-chloroperbenzoïque à 72%, puis purification par chromatographie sur gel de silice (éluant : éther diéthylique), 0,53 g (1,82 mmoles) du mélange diastéréoisomérique attendu est obtenu.
Rendement : 42% (mélange des deux diastéréoisomères)
Caractéristiques spectrales :
¹H RMN (DMSO) δ (ppm) : 1,30 (3H, s) ; 1,40 (3H, s) ; 2,50 (1H, m) ; 2,80 (1H, m) ; 3,10 (1H, m) ; 3,30 (7H, m) ; 3,40 (1H, m) ; 3,70 (2H, m) ; 3,80 (1H, m) ; 4,00 (1H, d) ; 4,70 (1H, d, échangeable par D₂O).

### Exemple 42: 5-O-Chloroacétylcarbamoyl-1,2-O-isopropylidène-3-[(2S)-(2:3-époxypropyl)]-4-O-méthyl-β-_{D}-fructopyranose et 5-O-chloroacétylcarbamoyl-1,2-O-isopropylidène-3-[(2R)-(2:3-époxypropyl)]-4-O-méthyl-β-_{D}-fructopyranose

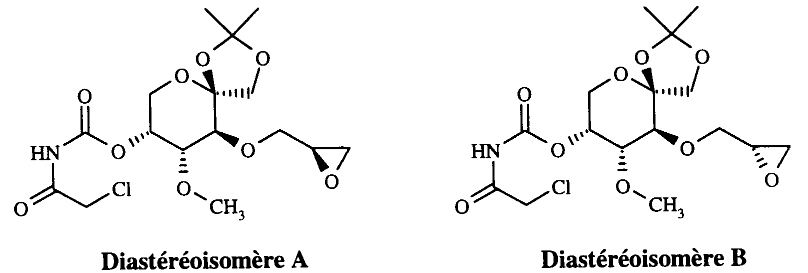

En procédant comme décrit pour la préparation de l'Exemple 3 à partir de 0,50 g (1,72 mmoles) du mélange de diastéréoisomères obtenus à l'Exemple 41 et de 0,25 ml (0,35 g ; 2,93 mmoles) d'isocyanate de chloroacétyle, puis purification par chromatographie (HPLC, colonne Kromasil 100-5C18, éluant: acétonitrile/eau, 3:7), 0,189 g (0,46 mmoles) de diastéréoisomère A attendu ainsi que 0,164 g (0,40 mmoles) de diastéréoisomère B, tous 2 sous la forme d'une mousse de couleur blanche sont obtenus.
Rendement : diastéréoisomère A : 27% diastéréoisomère B : 23%
Caractéristiques spectrales **Diastéréoisomère A :**
¹H RMN (DMSO ) δ (ppm) : 1,32 (3H, s) ; 1,40 (3H, s) ; 2,54 (1H, dxd) ; 2,72 (1H, t) ; 3,10 (1H, txd) ; 3,28 (3H, s) ; 3,29 (1H, m) ; 3,50 (1H, d) ; 3,60 (1H, dxd) ; 3,70 (1H, dxd) ; 3,85 (2H, d+s) ; 4,05 (2H, d+s) ;4,41 (2H, s) ; 5,29 (1H, s élargi) ; 11,01 (1H, s, échangeable par D₂O).
Caractéristiques spectrales **Diastéréoisomère B :**
¹H RMN (DMSO ) δ (ppm) : 1,32 (3H, s) ; 1,41 (3H, s) ; 2,60 (1H, m) ; 2,70 (1H, m) ; 3,10 (1H, m) ; 3,29 (3H, s) ; 3,50 (1H, d) ; 3,58 (1H, dxd) ; 3,70 (1H, dxd) ; 3,70 à 3,75 (2H, m) ; 3,80 à 3,85 (2H, m) ; 4,10 (1H, d) ; 4,42 (2H, s); 5,25 (1H, s élargi) ; 11,00 (1H, s, échangeable par D₂O).

| Analyse élémentaire : (Formule brute : C₁₆H₂₄ClNO₉ M=409,82) | | | | | |
|---|---|---|---|---|---|
| | | **C** | **H** | **N** | **Cl** |
| | **% calculé** | 46,89 | 5,90 | 3,42 | 8,65 |
| Diastéréoisomère A | **% trouvé** | 46,85 | 5,90 | 3,27 | 8,83 |
| Diastéréoisomère B | **% trouvé** | 47,33 | 5,96 | 3,32 | 8,59 |

### Exemple 43: 1,2-O-Isopropylidène-3-(5-méthylhex-1-ényl)-β-_{D}-psicopyranose

### Etape A :1,2:4,5-di-O-isopropylidène-3-(5-méthylhex-1-ényl)-β-_{D}-psicopyranose

A une solution de 6,55 ml de 5-méthylhex-1-yne (4,83 g ; 50,26 mmoles) dans 30 ml de tétrahydrofurane anhydre refroidie à-78°C sont ajoutés goutte-à-goutte et sous atmosphère d'azote 18 ml de n-butyllithium (2,5 molaire dans l'hexane soit 45,00 mmoles). L'ensemble est laissé sous agitation à -78°C pendant 15 minutes puis à 0°C pendant 30 minutes. Cette solution est alors additionnée goutte-à-goutte, à une solution de 1,2:4,5-di-O-isopropylidène-β-_{D}-*érythro*-2,3-hexodiulo-2,6-pyranose (décrit Exemple 1, Etape A ; 6,50 g ; 25,17 mmoles) dans 140 ml de toluène anhydre refroidie à -78°C. Après 2 heures d'agitation à -78°C le mélange réactionnel est versé dans une solution aqueuse à 10% de chlorure d'ammonium (150 ml) refroidie à 0°C. Le traitement habituel de la phase organique fournit 8,60 g de 1,2:4,5-di-O-isopropylidène-3-(5-méthylhex-1-ényl)-β-_{D}-psicopyranose sous la forme d'un résidu huileux que l'on utilise sans purification pour l'étape suivante.
L'hydrogénation d'une solution de 3,07 g (8,66 mmoles) de ce composé dans 120 ml de benzène en présence de 0,82 g de catalyseur de Lindlar pendant 1,5 heures conduit après filtration, évaporation et chromatographie sur gel de silice (éluant : pentane/acétate d'éthyle, 20:1) au composé attendu (2,41 g ; 6,76 mmoles) sous la forme d'une huile incolore.
Rendement : 75%

**Etape B :** 1,2-O-isopropylidène-3-(5-méthylhex-1-ényl)-β-_{D}-psicopyranose

En procédant selon le mode opératoire décrit pour la préparation de l'Exemple 1 (Etape C), à partir de 2,25 g (6,31 mmoles) du composé obtenu à l'étape précédente, puis évaporation des 25 ml du mélange acide acétique/eau, 4:1, 1,98 g (6,26 mmoles) de produit attendu sous la forme d'une huile incolore sont obtenus.
Rendement : 99%
Caractéristiques spectrales :
¹H RMN (DMSO) δ (ppm) : 0,85 (6H, d) ; 1,20 (2H, m) ; 1,30 (3H, s) ; 1,40 (3H, s) ; 1,51 (1H, o) ; 2,40 (2H txd) ; 3,50 (1H, dxd) ; 3,68 (1H, dxd) ; 3,81 (3H, m) ; 4,00 (1H, d) ; 4,58 (1H, d, échangeable par D₂O) ; 4,90 (1H, s, échangeable par D₂O) ; 5,15 (1H, d) ; 5,45 (1H, m) ; 5,75 (1H, d, échangeable par D₂O).

### Exemple 44: 1,2-O-Isopropylidène-3-(5-méthylhex-1-ényl]-4-O-méthyl-β-_{D}-psicopyranose

En procédant selon le mode opératoire décrit pour la préparation de l'Exemple 2, à partir de 1,98 g (6,26 mmoles) du composé obtenu à l'Exemple 43 et de 3,15 g de dibutylétain (12,65 mmoles) à reflux pendant 4 heures dans 60 ml de méthanol, puis en utilisant l'intermédiaire formé à l'issue de cette réaction et en le soumettant à l'action de 6,50 ml (14,82 g ; 104,41 mmoles) d'iodure de méthyle à reflux du dioxane (50 ml) pendant 37 heures, et après purification par chromatographie sur gel de silice (éluant : pentane/éther diéthylique, 3:1), 1,37 g (4,15 mmoles) de composé attendu sous la forme d'une mousse de couleur blanche sont obtenus.
Rendement : 66%
Caractéristiques spectrales :
¹H RMN (DMSO) δ (ppm) : 0,85 (6H, d) ; 1,18 (2H, m) ; 1,30 (3H, s) ; 1,40 (3H, s) ; 1,50 (1H, o) ; 2,40 (2H, m) ; 3,16 (1H, d) ; 3,30 (3H, s) ; 3,72 (1H, dxd) ; 3,82 (2H, m) ; 3,95 (1H, d) ; 4,06 (1H, m) ; 5,15 (1H, s, échangeable par D₂O) ; 5,18 (1H, d) ; 5,45 (1H, m) ; 5,90 (1H, d, échangeable par D₂O).

### Exemple 45: 1,2-O-Isopropylidène-3-[(2S,3S)-(3-isopentyloxiran-2-yl)]-4-O-méthyl-β-_{D}-psicopyranose et 1,2-O-isopropylidène-3-[(2R,3R)-(3-isopentyloxiran-2-yl)]-4-O-méthyl-β-_{D}-psicopyranose

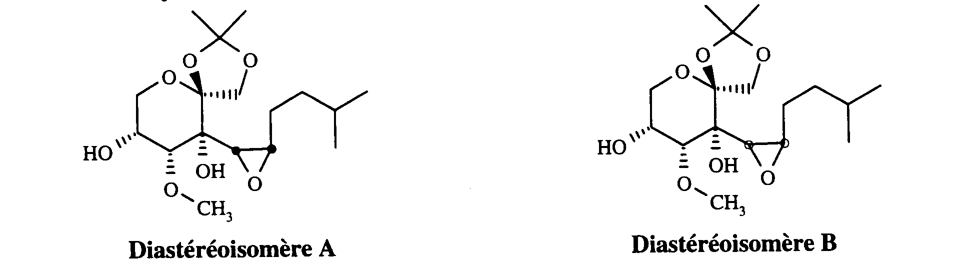

En procédant selon le mode opératoire décrit pour la préparation de l'Exemple 4, à partir de 1,31 g (3,96 mmoles) du composé obtenu à l'Exemple 44 et de 1,71 g ( 7,23 mmoles) d'acide 3-chloroperbenzoïque, puis purification par chromatographie sur gel de silice (éluant : éther diéthylique/pentane, 2:1), 0,69 g (1,99 mmoles) du diastéréoisomère A (Rf = 0,30) et 0,36 g (1,04 mmoles) du diastéréoisomère B (Rf = 0,10) tous 2 sous la forme d'une mousse de couleur blanche sont obtenus.
- Rendement :: diastéréoisomère A : 50%
diastéréoisomère B : 26%
Caractéristiques spectrales **Diastéréoisomère A :**
¹H RMN (DMSO ) δ (ppm) : 0,85 (6H, d) ; 1,28 (2H, m) ; 1,30 (3H, s) ; 1,40 (3H, s) ; 1,55 (1H, o) ; 1,80 (1H, m) ; 2,00 (1H, m) ; 2,80 (1H, txd) ; 2,90 (1H, d) ; 3,30 (1H, d) ; 3,35 (3H, s) ; 3,72 (1H, d) ; 3,75 (1H, m) ; 3,82 (1H, d) ; 4,10 (1H, s élargi) ; 4,40 (1H, d) ; 4,96 (1H, s, échangeable par D₂O) ; 5,93 (1H, d, échangeable par D₂O).
Caractéristiques spectrales **Diastéréoisomère B :**
¹H RMN (DMSO ) δ (ppm) : 0,85 (6H, d) ; 1,30 (2H, m) ; 1,30 (3H, s) ; 1,40 (3H, s) ; 1,55 (1H, o) ; 1,66 (1H, m) ; 1,76 (1H, m) ; 2,72 (1H, m) ; 2,90 (1H, d) ; 3,40 (3H, s) ; 3,45 (1H, d) ; 3,70 (1H, dxd) ; 3,80 (1H, dxd); 3,81 (1H, d) ; 4,02 (1H, m) ; 4,10 (1H, d) ; 4,95 (1H, s, échangeable par D₂O) ; 5,45 (1H, d, échangeable par D₂O).

### Exemple 46: 5-O-Chloroacétylcarbamoyl-1,2-O-isopropylidène-3-[(2S,3S)-(3-isopentyloxiran-2-yl)]-4-O-méthyl-β-_{D}-psicopyranose et 5-O-chloroacétylcarbamoyl-1,2-O-isopropylidène-3-[(2R,3R)-(3-isopentyloxiran-2-yl)]-4-O-méthyl-β-_{D}-psicopyranose

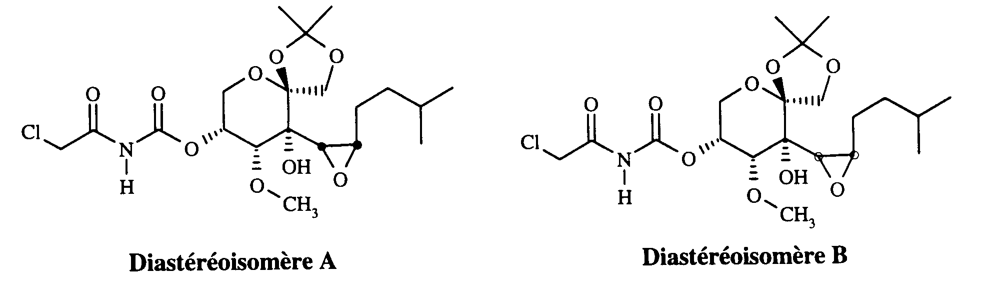

En procédant comme décrit pour la préparation de l'Exemple 3 à partir de 0,144 g (0,41 mmol) du diastéréoisomère A obtenu à l'Exemple 45 et de 0,06 ml (0,08 g ; 0,70 mmoles) d'isocyanate de chloroacétyle, puis purification par chromatographie sur gel de silice (éluant : éther diéthylique/pentane, 1:1), 0,175 g (0,37 mmoles) du dérivé attendu sous la forme d'un solide de couleur blanche sont obtenus.
Le diastéréoisomère B est obtenu selon la même procédure à partir du diastéréoisomère B obtenu à l'Exemple 45.
- Rendement :: diastéréoisomère A : 90%
diastéréoisomère B : 86%
Caractéristiques spectrales **Diastéréoisomère A :**
¹H RMN (DMSO) δ (ppm) : 0,85 (6H, d) ; 1,30 (5H, m) ; 1,40 (3H, s) ; 1,55 (1H, o) ; 1,80 (1H, m) ; 2,00 (1H, dxdxd) ; 2,80 (1H, m) ; 2,95 (1H, d) ; 3,30 (3H, s) ; 3,60 (1H, d) ; 3,80 (1H, d) ; 3,88 (1H, s, échangeable par D₂O) ; 3,98 (1H, d) ; 4,41 (2H, d) ; 4,50 (2H, s) ; 5,30 (1H, s élargi) ; 11,10 (1H,s, échangeable par D₂O).

| Analyse élémentaire : (Formule brute : C₂₀H₃₂ClNO₉ M=465,93) | | | | | |
|---|---|---|---|---|---|
| | | **C** | **H** | **N** | **Cl** |
| | **% calculé** | 51,56 | 6,92 | 3,01 | 7,61 |
| Diastéréoisomère A | **% trouvé** | 51,22 | 7,01 | 3,04 | 7,97 |
| Diastéréoisomère B | **% trouvé** | 51,47 | 7,00 | 3,03 | 8,27 |

### Exemple 47: 5-O-Ethoxycarbonylcarbamoyl-1,2-O-isopropylidène-3-[(2S*,3S*)-(3-isopentyloxiran-2-yl)]-4-O-méthyl-β-_{D}-psicopyranose

En procédant selon le mode opératoire décrit pour la préparation de l'Exemple 3, à partir de 0,15 g (0,43 mmoles) du diastéréoisomère A obtenu à l'Exemple 45 et de 0,06 ml (0,08 g ; 0,69 mmoles) d'isocyanate d'éthoxycarbonyle, puis purification par chromatographie sur gel de silice (éluant: pentane/diéthyléther, 1:1), 0,19 g (0,41 mmoles) de composé attendu sous la forme d'un solide de couleur blanche sont obtenus.
Rendement : 95%
Caractéristiques spectrales :
¹H RMN (DMSO) δ (ppm) : 0,86 (6H, d) ; 1,20 (3H, t) ; 1,32 (2H, m) ; 1,33 (3H, s) ; 1,35 (3H, s) ; 1,55 (1H, m) ; 1,70 à 2,10 (1H, m) ; 2,80 (1H, m) ; 2,95 (1H, dd) ; 3,35 (3H, s) ; 3,56 (1H, d) ; 3,76 (2H, m) ; 3,96 (3H, m, dont 1H échangeable par D₂O) ; 4,08 (2H, q) ; 4,43 (1H, d) ; 5,28 (1H, m) ; 10,62 (1H, s, échangeable par D₂O).

| Analyse élémentaire : (Formule brute : C₂₁H₃₅NO₁₀ M=461,51) | | | |
|---|---|---|---|
| | **C** | **H** | **N** |
| **% trouvé** | 54,61 | 7,52 | 2,94 |
| **% calculé** | 54,65 | 7,64 | 3,03 |

### Exemple 48: 5-O-Ethyloxycarbonylméthylcarbamoyl-1,2-O-isopropylidène-3-[(2S*,3S*)-(3-isopentyloxiran-2-yl)]4-O-méthyl-β-_{D}-psicopyranose

En procédant selon le mode opératoire décrit pour la préparation de l'Exemple 3, à partir de 0,15 g (0,43 mmoles) du diastéréoisomère A obtenu à l'Exemple 45 et de 0,06 ml (0,08 g; 0,66 mmoles) d'isocyanatoacétate d'éthyle, puis purification par chromatographie sur gel de silice (éluant : pentane/diéthyléther, 1:1), 0,17 g (0,36 mmoles) de composé attendu sous la forme d'un solide de couleur blanche sont obtenus.
Rendement : 83%
Caractéristiques spectrales :
¹H RMN (DMSO) δ (ppm) : 0,88 (6H, d) ; 1,21 (3H, t) ; 1,30 (2H, m) ; 1,35 (3H, s) ; 1,42 (3H, s) ; 1,56 (1H, m) ; 1,80 (1H, m) ; 1,95 (1H, m) ; 2,82 (1H, m) ; 2,98 (1H, d) ; 3,32 (3H, s) ; 3,55 (1H, d) ; 3,68 (2H, dd dont 1H échangeable par D₂O) ; 3,80 (3H, m) ; 3,94 (1H, d) ; 4,10 (2H, q) ; 4,43 (1H, d) ; 5,15 (1H, s élargi) ; 7,95 (1H, t, échangeable par D₂O).

| Analyse élémentaire : (Formule brute : C₂₂H₃₇NO₁₀ M=475,54) | | | |
|---|---|---|---|
| | **C** | **H** | **N** |
| **% trouvé** | 55,50 | 7,68 | 2,97 |
| **% calculé** | 55,57 | 7,84 | 2,95 |

### Exemple 49: 5-O-Chloroacétylcarbamoyl-1,2-O-isopropylidène-3-[(2S,3S)-(3-phénylpropyloxiran-2-yl)]-4-O-méthyl-β-_{D}-psicopyranose et 5-O-chloroacétylcarbamoyl-1,2-O-isopropylidène-3-[(2R,3R)-(3-phénylpropyloxiran-2-yl)]-4-O-méthyl-β-_{D}-psicopyranose

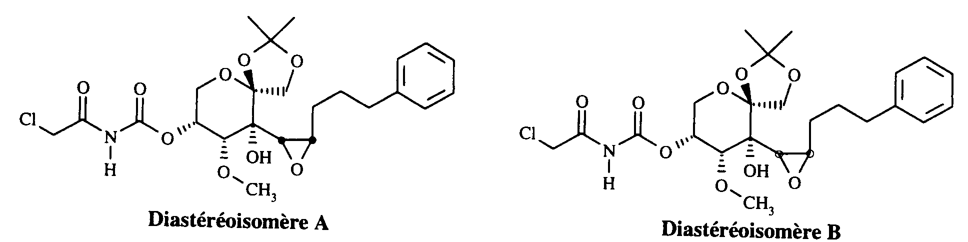

Ces deux diastéréoisomères sont obtenus en suivant les modes opératoires décrits aux Exemples 43 à 46, en remplaçant, dans l'Exemple 43 le 5-méthylhex-1-yne par le 5-phénylpent-1-yne.
Caractéristiques spectrales **Diastéréoisomère A :**
¹H RMN (DMSO) δ (ppm) : 1,35 (3H, s) ; 1,45 (3H, s) ; 1,75 (2H, m) ; 1,90 (1H, m) ; 2,05 (1H, txd) 2,60 (2H, t) ; 2,95 (1H, q) ; 3,00 (1H, s) ; 3,30 (3H, s) ; 3,60 (1H, d) ; 3,80 (2H, m, dont 1échangeable par D₂O) ; 3,90 (1H, s) ; 4,00 (1H, d) ; 4,50 (3H, m) ; 5,30 (1H, s élargi) 7,30 (5H, m); 11,01(1H, s, échangeable par D₂O).
Caractéristiques spectrales **Diastéréoisomère B :**
¹H RMN (DMSO ) δ (ppm) : 1,35 (3H, s) ; 1,45 (3H, s) ; 1,80 (3H, m) ; 2,00 (1H, m) ; 2,65 (2H, t) ; 2,85 (1H, m) ; 2,95 (1H, d) ; 3,35 (3H, s) ; 3,70 (1H, d) ; 3,80 (1H,d) ; 3,85 (1H,s) ; 4,00 (1H, d) ; 4,10 (2H, d, dont 1H échangeable par D₂O) ; 4,45 (2H, s) ; 5,25 (1H, s élargi) ; 7,30 (5H, m) ; 11,00 (1H, s, échangeable par D₂O).

| Analyse élémentaire : (Formule brute : C₂₄H₃₂ClNO₉ M=513,97) | | | | | |
|---|---|---|---|---|---|
| | | **C** | **H** | **N** | **Cl** |
| | **% calculé** | 56,09 | 6,28 | 2,73 | 6,90 |
| Diastéréoisomère A | **% trouvé** | 56,44 | 6,29 | 2,73 | 6,84 |
| Diastéréoisomère B | **% trouvé** | 56,55 | 6,49 | 2,53 | 6,78 |

### Exemple 50: 5-O-Chloroacétylcarbamoyl-3-désoxy-1,2-O-isopropylidène-3-(3-phénylpropyloxiran-2-yl)-4-O-méthyl-β-_{D}-fructopyranose

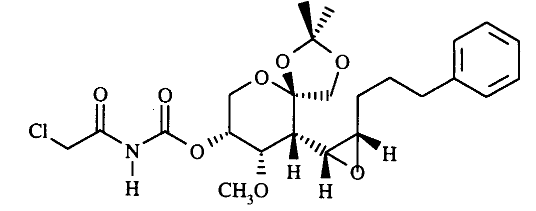

### Etape A : 1,2:4,5-di-O-isopropylidène-3-méthyloxalyl-3-(5-phénylpent-1-yne)-β-_{D}-fructopyranose

En remplaçant dans l'exemple 43 le 5-méthylhex-1-yne par le 5-phénylpent-1-yne on obtient le 1,2:4,5-di-O-isopropylidène-3-(3-phénylpropyloxiran-2-yl]-β-_{D}-psicopyranose attendu (1 seul isomère) avec un rendement de 88 % après chromatographie sur gel de silice (éluant : pentane/éther diéthylique, 2 : 1).
A une solution de 13,72 g (34,08 mmoles) de ce composé dans 250 ml de tétrahydrofurane anhydre refroidie à -78°C sont ajoutés goutte-à-goutte et sous atmosphère d'azote 44 ml de n-butyllithium (1,6 molaire dans l'hexane soit 70,40 mmoles). L'ensemble est placé sous agitation à -78°C pendant 1,5 heures et on y introduit goutte-à-goutte 9,20 ml (106,6 mmoles) de chlorure de méthyloxalyle. Le mélange réactionnel est laissé sous agitation et atmosphère d'azote pendant 1,75 heures et 30 minutes à température ambiante.
Le mélange est alors versé dans une solution aqueuse saturée de bicarbonate de sodium refroidie à 0°C (250 ml). Après traitement habituel de la phase organique on obtient 24 g d'un résidu huileux que l'on chromatographie sur gel de silice (éluant : chlorure de méthylène/acétate d'éthyle ; 99:1) pour donner 10,44 g (21,38 mmoles) du produit attendu sous forme d'huile incolore.
Rendement : 54% (2 étapes)
Caractéristiques spectrales :
¹H RMN (DMSO) δ (ppm) : de 1,10 à 1,50 (12H, 4 s); 1,72 (2H, q) ; 2,28 (2H, t) ; 2,70 (2H, t) ; 3,82 (3H, s) ; 3,90 (2H, m) ; 4,18 (1H, dd) ; 4,40 (2H, m) ; 4,78 (1H, d) ; 7,20 (3H, m) 7,30 (2H, m).

### Etape B : 1,2:4,5-di-O-isopropylidène-3-désoxy-3-(5-phénylpent-1-yne)-β-_{D}-fructopyranose

A une solution du composé décrit à l'étape A (2,50 g ; 5,11 mmoles) dans 70 ml de toluène anhydre sont ajoutés successivement 85 mg de 2,2'-azobis-(2-méthylpropionitrile) (0,52 mmoles) et 1,79 ml (6,65 mmoles) d'hydrure de tributylétain. L'ensemble est chauffé à reflux du toluène pendant 1 heure et on y additionne 0,25 ml (0,93 mmoles) d'hydrure de tributylétain supplémentaires. Après 1 heure de reflux supplémentaire le toluène est évaporé sous pression réduite et le résidu huileux résultant est dissous dans 70 ml d'éther diéthylique. On ajoute 30 ml d'une solution aqueuse de fluorure de potassium (3,6 molaire) puis on sépare les 2 phases. Après traitement habituel de la phase organique on obtient 4,29 g d'un résidu huileux que l'on chromatographie sur gel de silice (éluant : pentane/acétate d'éthyle ; 15:1) pour donner 0,62 g (1,60 mmoles) du produit attendu sous la forme d'une huile incolore.
Rendement : 31%

| Analyse Elémentaire : (Formule brute : C₂₃H₃₀O₅ M=386,49) | | |
|---|---|---|
| | **C** | **H** |
| **% trouvé** | 71,04 | 7,90 |
| **% calculé** | 71,48 | 7,82 |

### Etape C : 5-O-hloroacétylcarbamoyl-3-désoxy-1,2-O-isopropylidène-3-(3-phénylpropyloxiran-2-yl)4-O-méthyl-β-_{D}-fructopyranose

L'hydrogénation d'une solution de 0,62 g (1,60 mmoles) du composé décrit à l'étape B dans 10 ml de benzène en présence de 0,29 g de catalyseur de Lindlar pendant 45 minutes conduit après filtration et évaporation au composé attendu (0,60 g; 1,54 mmoles) sous la forme d'une huile de couleur jaune que l'on utilise sans purification pour l'étape suivante (Rendement brut: 96%). En procédant selon les protocoles expérimentaux décrits aux exemples 1 (étape C) à 4 à partir du composé décrit ci-dessus on obtient le 5-O-chloroacétylcarbamoyl-3-déshydroxy-1,2-O-isopropylidène-3-[(5'-phényl)-pentyloxiran-1'-yl]-4-O-méthyl-β-_{D}-fructopyranose sous forme de mousse de couleur blanche.

| Analyse Elémentaire : (Formule brute : C₂₄H₃₂ClNO₈ M=497,97) | | | | |
|---|---|---|---|---|
| | **C** | **H** | **N** | **Cl** |
| **% trouvé** | 57,53 | 6,52 | 2,78 | 7,07 |
| **% calculé** | 57,89 | 6,48 | 2,81 | 7,12 |

### Exemple 51: 3,5-di-O-Chloroacétylcarbamoyl-1,2-O-isopropylidène-3-(3-phénylpropyloxiran-2-yl]-4-O-méthyl-β-_{D}-psicopyranose

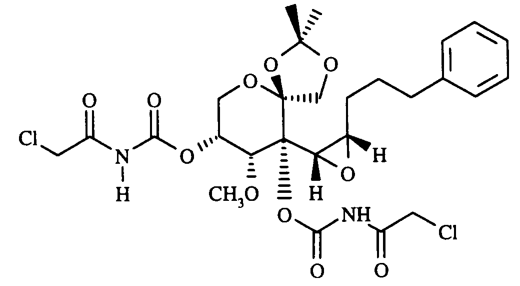

A une solution de 0,61 g (1,19 mmoles) du diastéréoisomère A de l'exemple 49 dans 7 ml de tétrahydrofurane anhydre sont ajoutés, à température ambiante et sous atmosphère d'azote, 0,50 ml (5,87 mmoles) d'isocyanate de chloroacétyle. Le mélange réactionnel est chauffé à reflux du tétrahydrofurane pendant 20 heures et on ajoute 0,20 ml d'isocyanate de chloroacétyle supplémentaires. L'ensemble est à nouveau porté à reflux pendant 24 heures puis la solution réactionnelle est versée dans 5 ml d'eau glacée. Après une heure d'agitation et traitement habituel de la phase organique on obtient un résidu huileux que l'on chromatographie sur gel de silice (éluant : éther diéthylique) pour donner 0,42 g (0,66 mmoles) de produit attendu sous la forme d'une mousse de couleur blanche.
Rendement : 55%

| Analyse Elémentaire : (Formule brute : C₂₇H₃₄Cl₂N₂O₁₁ M=633,48) | | | | |
|---|---|---|---|---|
| | **C** | **H** | **N** | **Cl** |
| **% trouvé** | 51,82 | 5,74 | 4,04 | 10,88 |
| **% calculé** | 51,19 | 5,41 | 4,42 | 11,19 |

### Exemple 52: 3,5-di-O-Chloroacétylcarbamoyl-1,2-O-isopropylidène-4-O-méthyl-3-phénylpentyl-β-_{D}-psicopyranose

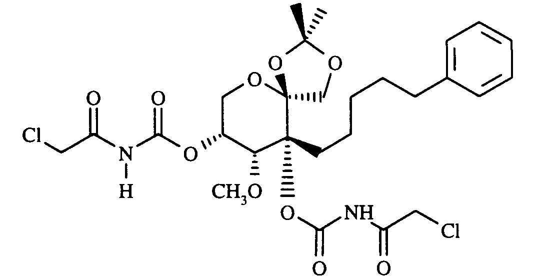

En procédant selon le mode opératoire décrit pour la préparation des exemples 43 et 44 en remplaçant le 5-méthylhex-1-yne par le 5-phénylpent-1-yne on obtient le 1,2-O-isopropylidène-3-(phénylpent-1-ényl)4-O-méthyl-β-_{D}-psicopyranose attendu. L'hydrogénation de 1,97 g (5,23 mmoles) de ce composé en présence de 0,90 g de Palladium sur charbon (10%) dans 100 ml d'acétate d'éthyle conduit au 1,2-O-isopropylidène-3-phénylpentyl-4-O-méthyl-β-_{D}-psicopyranose. Ce composé (0,55 g; 1,44 mmoles) est mis en solution dans 9 ml de dichlorométhane en présence de 0,27 ml (3,17 mmoles) d'isocyanate de chloroacétyle suivant le mode opératoire décrit pour la préparation de l'exemple 3. On obtient après chromatographie sur gel de silice (éluant : éther diéthylique/pentane ; 2:1) 0,74 g (1,19 mmoles) du produit attendu sous forme de mousse de couleur blanche.
Rendement (dernière étape) : 82%

| Analyse Elémentaire : (Formule brute : C₂₇H₃₆Cl₂N₂O₁₀ M=619,50) | | | | |
|---|---|---|---|---|
| | **C** | **H** | **N** | **Cl** |
| **% trouvé** | 52,72 | 5,88 | 4,35 | 11,34 |
| **% calculé** | 52,35 | 5,86 | 4,52 | 11,45 |

### Exemple 53: 3,5-di-O-Chloroacétylcarbamoyl-3-isopropyl-1,2-O-isopropylidène-4-O-méthyl-β-_{D}-psicopyranose

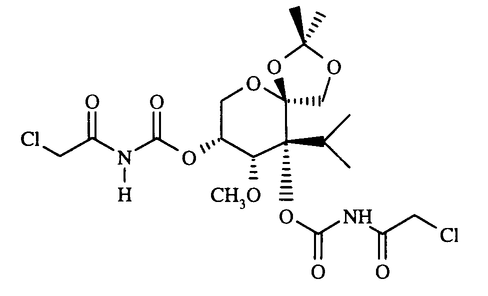

A une solution de 1,35 g (4,92 mmoles) de 1.2-O-isopropylidène-3-isopropényl-4-O-méthyl-β-_{D}-psicopyranose (exemple 2) dans 50 ml d'acétate d'éthyle sont ajoutés 1,00 g de Palladium sur charbon (10%). Le mélange réactionnel est placé sous agitation et atmosphère d'hydrogène pendant 20 heures puis filtré. Après évaporation, on isole quantitativement le produit brut sous forme d'une huile. Ce composé (1,35 g ; 4,88 mmoles) est mis en solution dans 30 ml de dichlorométhane en présence de 1,25 ml (14,67 mmoles) d'isocyanate de chloroacétyle, suivant le mode opératoire décrit pour la préparation de l'exemple 3. On obtient, après chromatographie sur gel de silice (éluant : pentane/acétate d'éthyle ; 2:1), 1,84 g (3,57 mmoles) du produit attendu sous forme d'une mousse de couleur blanche.
Rendement (dernière étape) : 73%

| Analyse Elémentaire : (Formule brute : C₁₉H₂₈Cl₂N₂O₁₀ M=515,34) | | | | |
|---|---|---|---|---|
| | **C** | **H** | **N** | **Cl** |
| **% trouvé** | 43,92 | 5,57 | 5,25 | 13,44 |
| **% calculé** | 44,28 | 5,48 | 5,44 | 13,76 |

### Exemple 54: 3,5-di-O-Chloroacétylcarbamoyl-1,2-O-isopropylidène-3-(2-méthyloxiran-2-yl)-4-O-méthyl-β-_{D}-psicopyranose

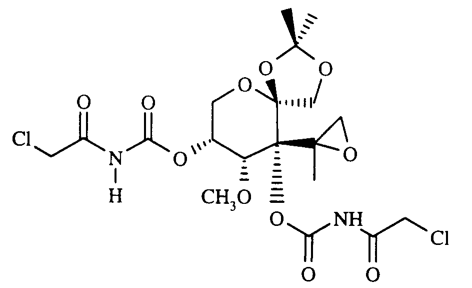

A une solution de 1,50 g (3,66 mmoles) du composé décrit exemple 4 dans 15 ml de tétrahydrofurane anhydre sont ajoutés, à température ambiante et sous atmosphère d'azote, 1,55 ml (18,20 mmoles) d'isocyanate de chloroacétyle. Le mélange réactionnel est chauffé à reflux du tétrahydrofurane pendant 21 heures puis la solution réactionnelle est versée dans 10 ml d'eau glacée. Après une heure d'agitation et traitement habituel de la phase organique, on obtient un résidu huileux que l'on chromatographie en HPLC sur gel de silice greffée (RP 18 ; éluant : acétonitrile/eau ; 40:60) pour donner 0,47 g (0,89 mmoles) de produit attendu sous forme d'une mousse de couleur blanche.
Rendement : 24%

| Analyse Elémentaire : (Formule brute : C₁₉H₂₆Cl₂N₂O₁₁ M=529,33) | | | | |
|---|---|---|---|---|
| | **C** | **H** | **N** | **Cl** |
| **% trouvé** | 42,78 | 4,84 | 4,80 | 16,48 |
| **% calculé** | 42,01 | 4,85 | 5,09 | 16,06 |

### Exemple 55: 1,2-Carbonate-5-O-chloroacétylcarbamoyl-4-O-méthyl-3-phénylpentyl-β-_{D}-psicopyranose

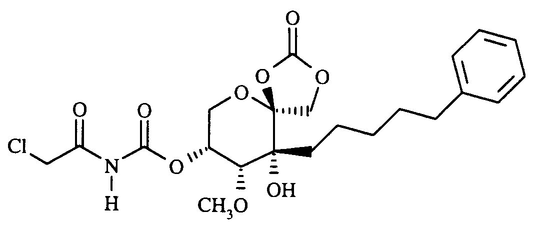

### Etape A : 1,2-O-isopropylidène-5-O-benzyl-4-O-méthyl-3-[(5-phénylpent-1-ényl)-β-_{D}-psicopyranose

En procédant selon le mode opératoire décrit pour la préparation des exemples 43 et 44 en remplaçant le 5-méthylhex-1-yne par le 5-phénylpent-1-yne on obtient le 1,2-O-isopropylidène-3-(5-phénylpent-1-ényl) attendu. A une solution de 2,01 g (5,31 mmoles) de ce composé dans 10 ml de tétrahydrofurane anhydre sont ajoutés à 0°C 0,28 g d'hydrure de sodium (60% dans l'huile) et 0,20 g (0,54 mmoles) d'iodure de tétrabutylammonium. Après 30 minutes d'agitation à 0°C on additionne 0,76 ml (6,39 mmoles) de bromure de benzyle et on laisse le mélange réactionnel sous agitation à température ambiante pendant 19 heures.
Après traitement habituel de la phase organique et chromatographie sur gel de silice (éluant : pentane/ éther diéthylique ; 4:1), 2,30 g (4,91 mmoles) de composé attendu sous forme d'huile incolore sont obtenus.
Rendement : 92%
Caractéristiques spectrales :
¹H RMN (DMSO) δ (ppm) : 1,28 (3H, s) ; 1,38 (3H, s) ; 1,60 (2H, m) ; 2,40 (2H, m) ; 2,55 (2H, t) ; 3,30 (3H, s) ; 3,39 (1H, s élargi) ; 3,75 (1H, dd) ; 3,82 (1H, d) ; 3,95 (1H, dd) ; 4,00 (1H, d) ; 4,03 (1H, s élargi) ; 4,48 (1H, s, échangeable par D₂O) ; 4,61 (1H, d) ; 4,71 (1H, d) ; 5,25 (1H, d) ; 5,52 (1H, m) ; de 7,10 à 7,40 (10H, m).

### Etape B : 1,2-carbonate-5-O-benzyl-4-O-méthyl-3-(5-phénylpent-1-ényl)-β-_{D}-psicopyranose

En procédant selon le mode opératoire décrit pour la préparation de l'exemple 9 à partir de 1,75 g (3,73 mmoles) de composé décrit à l'étape précédente et de 18 g de résine acide (DOWEX 50X8-100) dans 60 ml d'un mélange tétrahydrofurane/eau ; 1:1 on obtient 1,19 g (2,77 mmoles 74%) de produit brut sous forme d'une huile de couleur jaune. A une solution de 1,10 g (2,57 mmoles) de ce composé dans 20 ml de tétrahydrofurane anhydre sont ajoutés par portions de 1,50 g et sur une période de 72 heures, en chauffant à reflux, 5,50 g (33,91 mmoles) de N,N-carbonyldiimidazole.
Le mélange réactionnel est alors dilué à l'éther diéthylique puis lavé à l'aide d'une solution molaire d'acide chlorhydrique. La phase organique est ensuite neutralisée au bicarbonate de sodium puis séchée sur sulfate de magnésium et évaporée. On obtient un résidu huileux que l'on chromatographie sur gel de silice (éluant : pentane/ éther diéthylique ; 3:1), 0,80 g (1,77 mmoles) de composé attendu sous forme d'une mousse blanche sont obtenus.
Rendement : 51% (2 étapes)
Caractéristiques spectrales :
¹H RMN (DMSO) δ (ppm) : 1,61 (2H, m) ; 2,40 (2H, m) ; 2,58 (2H, t) ; 3,45 (3H, s) ; 3,55 (1H, s) ; 3,75 (2H, m) ; 3,80 (1H, m) ; 4,22 (1H, d) ; 4,30 (1H, d) ; 4,55 (2H, s) ; 5,15 (1H, d); 5,33 (1H, s, échangeable par D₂O) ; 5,60 (1H, m) ; de 7,15 à 7,40 (10H, m).

### Etape C : 1,2-carbonate-5-O-chloroacétylcarbamoyl-4-O-méthyl-3-phénylpentyl-β-_{D}-psicpyranose

A une solution de 0,70 g (1,55 mmoles) de 1,2-carbonate-5-O-benzyl-4-O-méthyl-3-(5-phénylpent-1-ényl)-β-_{D}-psicopyranose décrit à l'étape précédente dans 40 ml d'acétate d'éthyle sont ajoutés 0,40 g de Palladium sur charbon (10%).
Le mélange réactionnel est placé sous agitation et atmosphère d'hydrogène pendant 4 heures puis filtré. Après évaporation, on isole quantitativement ce produit brut. Ce composé (0,48 g ; 1,31 mmoles) est mis en solution dans 10 ml de dichlorométhane en présence de 0,39 ml (4,58 mmoles) d'isocyanate de chloroacétyle à 0° C pendant 3 heures. Après traitement habituel de la phase organique et chromatographie sur gel de silice (éluant : heptane/acétate d'éthyle ; 5:2), 0,32 g (0,66 mmoles) de composé attendu, sous forme de mousse blanche, sont obtenus.
Rendement : 50%

| Analyse Elémentaire : (Formule brute : C₂₂H₂₈ClNO₉ M=485,92) | | | | |
|---|---|---|---|---|
| | **C** | **H** | **N** | **Cl** |
| **% trouvé** | 54,98 | 5,85 | 2,90 | 7,66 |
| **% calculé** | 54,38 | 5,81 | 2,88 | 7,30 |

### Exemple 56 : 1,2-Carbonate-3,5-di-O-chloroacétylcarbamoyl-4-O-méthyl-3-phénylpentyl-β-_{D}-psicopyranose

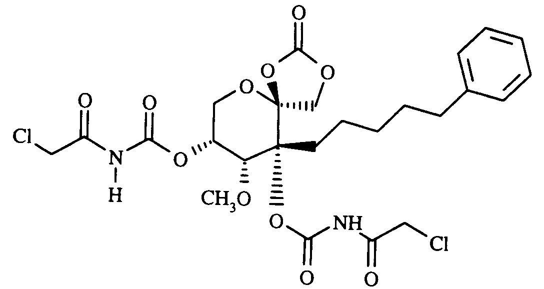

A une solution de 1,2-carbonate-5-O-chloroacétylcarbamoyl-4-O-méthyl-3-phénylpentyl-β-_{D}-psicpyranose (0,21 g ; 0,43 mmoles) dans 6 ml de dichlorométhane anhydre sont ajoutés 0,13 ml (1,53 mmoles) d'isocyanate de chloroacétyle à température ambiante. Le mélange réactionnel est placé sous agitation et atmosphère d'azote pendant 49 heures.
Après traitement habituel de la phase organique et chromatographie sur gel de silice (éluant : pentane/acétate d'éthyle ; 1:1), 0,22 g (0,36 mmoles) de composé attendu sous forme de mousse blanche sont obtenus.
Rendement : 84%

| Analyse Elémentaire : (Formule brute : C₂₅H₃₀Cl₂N₂O₁₁ M=605,43) | | | | |
|---|---|---|---|---|
| | **C** | **H** | **N** | **Cl** |
| **% trouvé** | 49,88 | 5,01 | 4,28 | 11,47 |
| **% calculé** | 49,60 | 4,99 | 4,63 | 11,71 |

### ETUDE PHARMACOLOGIQUE

### Exemple A: Cytotoxicité des composés et des produits de référence

Trois lignées cellulaires ont été utilisées :
- 1 leucémie murine, L1210,
- 1 carcinome épidermoïde humain, A431,
- 1 culture primaire de cellules endothéliales d'aorte de porc, CEAP.

Les cellules sont cultivées dans du milieu de culture RPMI 1640 complet contenant 10% de sérum de veau foetal, 2 mM de glutamine, 50 unités/ml de pénicilline, 50 µg/ml de streptomycine et 10 mM d'HEPEs (pH = 7,4).

Les cellules sont réparties dans des microplaques et exposées aux composés cytotoxiques. Les cellules sont ensuite incubées pendant deux jours (L1210), 3 jours (CEAP) et 4 jours (A431).
Le nombre de cellules viables est ensuite quantifié par un essai colorimétrique, le Microculture Tetrazolium Assay (Carmichael J., DeGraff W. G., Gazdar A. F., Minna J. D. and Mitchell J. R., Evaluation of a tetrazolium-based semiautomated colorimetrie assay : assessment of chemosensitivity testing, *Cancer Res.*, 47, 936-942, (1987)).

Les composés de la présente invention ont montré un pouvoir cytotoxique très important sur ces trois lignées cellulaires.
A titre d'exemple, les IC₅₀ (concentrations en cytotoxique qui inhibent à 50 % la prolifération des cellules traitées) sont selon les lignées cellulaires, de 3 à 10 fois inférieures à celles de la fumagilline.

### Exemple B: Inhibition de la néovascularisation de la membrane chorio-allantoïdienne d'embryon de poulet

Ce test est réalisé avec des embryons de poulet comme décrit précédemment (Crum R., Szabo S. and Folkman J., Science, (1985), 230, 1375-1378). Les oeufs fécondés (J0) sont incubés à 37°C. Une poche d'air est créée en prélevant 1 ml d'albumine (J3), puis une fenêtre est découpée dans la coquille (J4) et la membrane vitelline est enlevée pour dégager la membrane chorio-allantoïdienne (MCA).
Les produits à tester sont solubilisés dans de l'éthanol et déposés sur des disques de méthylcellulose qui sont séchés et déposés sur la MCA 48 h. plus tard (J6). Entre 8 et 16 oeufs sont utilisés par groupe. La zone située autour du disque est ensuite examinée 48 heures plus tard. Les oeufs présentant une zone avasculaire supérieure à 4 mm de diamètre sont dénombrés et les résultats sont exprimés en pourcentage d'oeufs présentant une zone avasculaire. Les résultats obtenus sont présentés dans le tableau suivant:

| ***Inhibition de la néovascularisation de la membrane chorio-allantoïdienne d'embryon de poulet*** *(dose 125 nM)* | |
|---|---|
| **Exemple** | **%** |
| 3 | 59 ± 6 |
| 4 | 84 ± 4 |
| 6 | 51 ± 5 |
| 8 | 55 |
| 13 | 55 ± 3 |
| 17 | 48 ± 12 |
| 20 | 59 ± 6 |
| 22 | 57 ± 12 |
| 42 (Diast. A) | 53 ± 13 |
| 46 (Diast. A) | 68 ± 12 |
| 46 (Diast. B) | 75 ± 13 |
| 49 (Diast. A) | 76 ± 7 |
| 49 (Diast. B) | 79 ± 7 |
| 50 | 75 ± 8 |
| 52 | 88 ± 5 |
| 53 | 95 ± 5 |
| 55 | 90 |
| 56 | 60 |
| *Fumagilline* | *80* |

### Exemple C: Composition pharmaceutique : comprimés

### Formule de préparation pour 1000 comprimés dosés à 50 mg.

| | |
|---|---|
| Composé de l'exemple 49 | 50 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications

1. Composés de formule (I) : 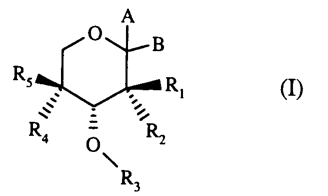 dans laquelle :
**A** soit représente -OR₆ et **B** représente -CH₂-X,
soit forme avec **B** et l'atome de carbone qui les porte un hétérocycle oxygéné choisi parmi l'oxirane, le 2,2-diméthyl[1,3]dioxolane, et la [1,3]dioxolan-2-one,
**R₁**
- soit représente le radical : 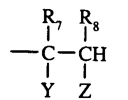 dans lequel Y et Z, soit représentent chacun l'hydrogène, soit forment ensemble une double liaison, ou forment ensemble, avec les atomes de carbone qui les portent, un cycle oxirane,
et **R₂** est choisi parmi l'hydrogène, le radical hydroxy et le radical -OR₉,
- soit le radical : 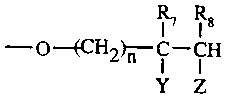 dans lequel n prend les valeurs entières 1 à 4, bornes incluses, et Y et Z, soit représentent chacun l'hydrogène, soit forment ensemble une double liaison, ou forment ensemble, avec les atomes de carbone qui les portent, un cycle oxirane,
et **R₂** représente l'hydrogène,
- soit **R₁** représente l'hydrogène,
et **R₂** représente le radical: 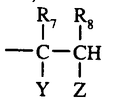 tel que défini ci-dessus,
**R₃** est choisi parmi l'hydrogène, un radical alkyle linéaire ou ramifié comportant de 1 à 6 atomes de carbone et éventuellement substitué, le radical benzyle éventuellement substitué, le radical benzoyle éventuellement substitué, le radical formyle éventuellement substitué, un radical acyle, éventuellement insaturé, linéaire ou ramifié comportant de 1 à 6 atomes de carbone et éventuellement substitué, le radical allyle, et le radical carbamoyle éventuellement mono- ou di-substitué,
**R₄**
- soit est choisi parmi le radical hydroxy, un radical alkoxy linéaire ou ramifié comportant de 1 à 6 atomes de carbone et éventuellement substitué, le radical carbamoyloxy éventuellement mono- ou di-substitué, le radical pipérazinylcarbonyloxy substitué en position 4 par le radical R₉ et le radical imidazol-1-yl-carbonyloxy,
et **R₅** représente l'hydrogène,
- soit forme avec **R₅** et l'atome de carbone qui les porte un groupement carbonyle,
**R₆, R₇** et **R₈** sont choisis indépendamment l'un de l'autre parmi l'hydrogène, un radical alkyle linéaire ou ramifié comportant de 1 à 6 atomes de carbone et éventuellement substitué, et un radical phénylalkyle dans lequel le groupement phényle est éventuellement substitué et le groupement alkyle, linéaire ou ramifié et éventuellement substitué, comporte de 1 à 6 atomes de carbone,
**R₉** est choisi parmi l'hydrogène, un radical alkyle linéaire ou ramifié comportant de 1 à 6 atomes de carbone et éventuellement substitué, le radical formyle éventuellement substitué, un radical acyle, éventuellement insaturé, linéaire ou ramifié comportant de 1 à 6 atomes de carbone et éventuellement substitué, un radical alkoxycarbonyle linéaire ou ramifié comportant de 1 à 6 atomes de carbone et éventuellement substitué, le radical benzyle éventuellement substitué, et le radical carbamoyle éventuellement mono- ou di-substitué,
**X** est choisi parmi le radical hydroxy, un atome d'halogène, un radical phénylsulfonyloxy éventuellement substitué et un radical alkylsulfonyloxy linéaire ou ramifié, éventuellement substitué et comportant de 1 à 6 atomes de carbone,
leurs éventuels isomères optiques et géométriques sous forme pure ou en mélange, ainsi que leurs éventuels sels d'addition à un acide, pharmaceutiquement acceptables,
étant entendu que :
- le terme "éventuellement mono- ou di-substitué" associé aux radicaux carbamoyle et carbamoyloxy ci-dessus définis signifie que un seul ou les deux atomes d'hydrogène portés par l'atome d'azote peut ou peuvent être substitués (et de manière indépendante, lorsque les deux atomes d'hydrogène sont substitués) par :
- un radical alkyle linéaire ou ramifié comportant de 1 à 6 atomes de carbone, et éventuellement substitué,
- le radical formyle éventuellement substitué,
- un radical acyle linéaire ou ramifié, comportant de 1 à 6 atomes de carbone, éventuellement insaturé et éventuellement substitué,
- le radical benzoyle, éventuellement substitué,
- le radical phényle, éventuellement substitué,
- le radical naphtyle, éventuellement substitué, et
- un radical amino éventuellement substitué par un ou deux radicaux alkyles comportant de 1 à 6 atomes de carbone en chaîne linéaire ou ramifiée et chacun d'eux pouvant être éventuellement substitués,
- le terme "éventuellement substitué" associé aux radicaux alkyle, alkoxy, alkoxycarbonyle, formyle, acyle, benzyle, benzoyle, phényle et naphtyle signifie que ces radicaux peuvent être substitués par une ou plusieurs entités chimiques choisies parmi hydroxy, halogène, trihalogénométhyle, amino, alkylamino, dialkylamino, alkoxy linéaire ou ramifié comportant de 1 à 6 atomes de carbone, alkoxycarbonyle linéaire ou ramifié comportant de 1 à 6 atomes de carbone et acyle linéaire ou ramifié comportant de 1 à 6 atomes de carbone,
- le terme éventuellement substitué associé aux radicaux alkylsulfonyloxy et phénylsulfonyloxy signifie que ces radicaux peuvent être substitués par un ou plusieurs radicaux alkyles linéaires ou ramifiés et comportant de 1 à 6 atomes de carbone,
- par radical acyle insaturé, on entend plus particulièrement les radicaux acryloyle et méthacryloyle.

2. Composés selon la revendication 1 pour lesquels A et B forment ensemble, et avec l'atome de carbone qui les porte, le cycle 2,2-diméthyl[1,3]dioxolane,
leurs éventuels isomères optiques et géométriques sous forme pure ou en mélange, ainsi que leurs éventuels sels d'addition à un acide, pharmaceutiquement acceptables.

3. Composés selon la revendication 1 pour lesquels A et B forment ensemble, et avec l'atome de carbone qui les porte, le cycle oxirane,
leurs éventuels isomères optiques et géométriques sous forme pure ou en mélange, ainsi que leurs éventuels sels d'addition à un acide, pharmaceutiquement acceptables.

4. Composé selon la revendication 1 qui est le 5-O-chloroacétylcarbamoyl- 1,2-O-isopropylidène-3-(3-phénylpropyloxiran-2-yl)-4-O-méthyl-β-_{D}-psicopyranose, et ses isomères optiques.

5. Composé selon la revendication 1 qui est le 5-O-chloroacétylcarbamoyl-1,2-O-isopropylidène-3- [(2*S*,3*S*)-(3-phényl-propyloxiran-2-yl)]-4-O-méthyl-β-_{D}-psicopyranose.

6. Composé selon la revendication 1 qui est le 5-O-chloroacétylcarbamoyl-1,2-O-isopropylidène-3-[(2*R*,3*R*)-(3-phényl-propyloxiran-2-yl)]-4-O-méthyl-β-_{D}-psicopyranose.

7. Composé selon la revendication 1 qui est le 5-O-chloroacétylcarbamoyl-1,2-O-isopropylidène-3-(2-méthyloxiran-2-yl)-4-O-méthyl-β-_{D}-psicopyranose,
et son isomère optique.

8. Composé selon la revendication 1 qui est le 1-bromo-1-désoxy-3-(2-méthyloxiran-2-yl)- 4-O-méthyl-5-O-méthylcarbamoyl-β-_{D}-psicopyranose,
et son isomère optique.

9. Composé selon la revendication 1 qui est le 5-O-{1-[4-((2,3,4-triméthoxybenzyl)pipérazinyl)]carbonyl}-1,2-O-isopropylidène-3-(2-méthyloxiran-2-yl)-4-O-méthyl-β-_{D}-psicopyranose,
et son isomère optique.

10. Composé selon la revendication 1 qui est le 4,5-di-O-chloroacétylcarbamoyl-1,2-O-isopropylidène-3-(2-méthyloxiran-2-yl)-β-_{D}-psicopyranose,
et son isomère optique.

11. Composé selon la revendication 1 qui est le (1*S*,6*R*,7*R*,8*R*)-8-isopropényl-6,7-diméthoxy- 1 ,4-dioxaspiro[2,5]octan-8-ol.

12. Composé selon la revendication 1 qui est le 5-O-chloroacétylcarbamoyl-1,2-O-isopropylidène-3-[(2*S*)-(2: 3-époxypropyl)]-4-O-méthyl-β-_{D}-fructopyranose,
et son isomère optique.

13. Composé selon la revendication 1 qui est le 5-O-chloroacétylcarbamoyl-1,2-O-isopropylidène-3-(3-isopentyloxiran-2-yl)-4-O-méthyl-β-_{D}-psicopyranose,
et ses isomères optiques.

14. Procédé de préparation des composés de formule (I) selon la revendication 1, caractérisé en ce que l'on soumet le 1,2:4,5-di-O-isopropylidène-β-_{D}-fructopyranose de formule (II) : 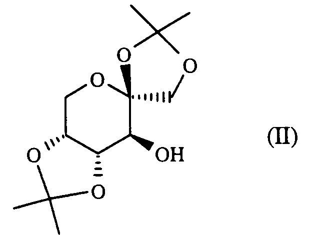 préparé selon le mode opératoire décrit par E.J. Prisbe *et al.* (*J. Org. Chem.*, *41*, (1976), 1836-1846).
**soit** : à un oxydant, de manière à obtenir la cétone de formule (III) : 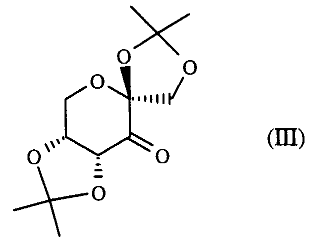 qui est soumise à l'action d'un composé de formule (IVa) préparé à partir de l'halogénure vinvliaue correspondant : 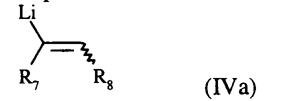 dans laquelle R₇ et R₈ sont choisis indépendamment l'un de l'autre parmi l'hydrogène, un radical alkyle linéaire ou ramifié comportant de 1 à 6 atomes de carbone, et éventuellement substitué, et un radical phénylalkyle dans lequel le groupement phényle est éventuellement substitué et le groupement alkyle, linéaire ou ramifié et éventuellement substitué, comporte de 1 à 6 atomes de carbone,
afin d'obtenir le composé de formule (Va₁) : 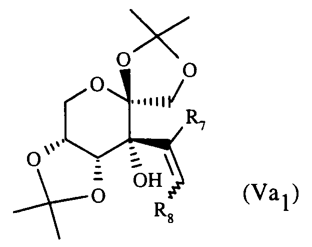 dans laquelle R₇ et R₈ sont tels que définis précédemment,
qui peut être éventuellement soumis à l'action d'un halogénure, d'alkyle, d'un halogénure d'acyle, d'un halogénure de benzyle, d'un halogénoformiate d'alkyle, ou d'un isocyanate afin de conduire au composé de formule (Va₂) : 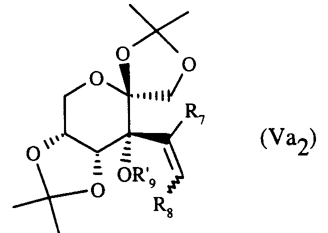 dans laquelle R₇ et R₈ sont tels que définis précédemment et R'₉ est choisi parmi un radical alkyle linéaire ou ramifié contenant de 1 à 6 atomes de carbone et éventuellement substitué, le radical formyle éventuellement substitué, un radical acyle contenant de 1 à 6 atomes de carbone et éventuellement substitué, le radical benzyle éventuellement subtitué, un radical alkoxycarbonyle linéaire ou ramifié contenant de 1 à 6 atomes de carbone et éventuellement substitué, et un radical carbamoyle éventuellement mono- ou di-substitué,
composé de formule (Va₁) qui peut éventuellement être soumis à l'action de chlorure de méthyloxalyle, en présence de n-butyllithium, de façon à obtenir le composé de formule (Va₃) : 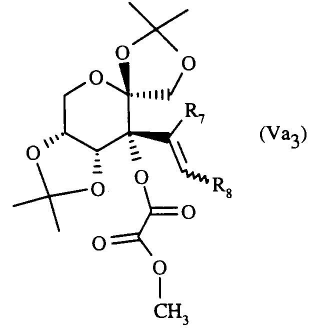 dans laquelle R₇ et R₈ sont tels que définis précédemment,
qui est ensuite soumis à l'action d'hydrure de tributylétain pour conduire au composé de formule (Va₄) : 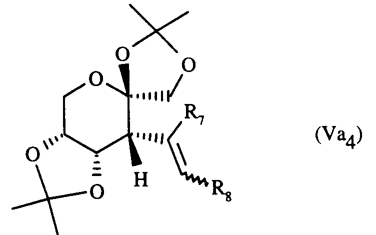 dans laquelle R₇ et R₈ sont tels que définis précédemment,
l'ensemble des composés de formule (Va₁) et (Va₂) formant le composé de formule (Va) : 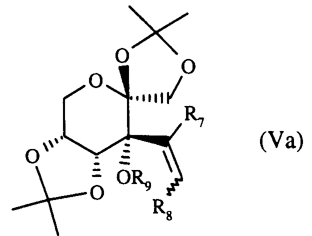 dans laquelle R₇ et R₈ sont tels que définis précédemment et R₉ est choisi parmi l'hydrogène et le radical R'₉ tel que défini précédemment,
**soit** : directement à l'action d'un composé de formule (IVb) : 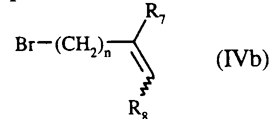 dans laquelle R₇ et R₈ sont tels que définis précédemment, et n prend les valeurs entières 1 à 4, bornes incluses,
afin d'obtenir le composé de formule (Vb) : 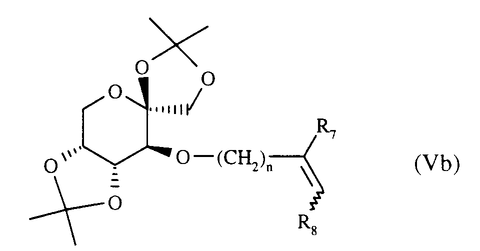 dans laquelle R₇, R₈ et n sont tels que définis précédemment,
les composés de formules (Va₄), (Va) et (V_{b}) étant ensuite hydrolysés, en milieu acide, pour conduire respectivement aux diols de formule (VIa₄), (VIa) et (VIb) : 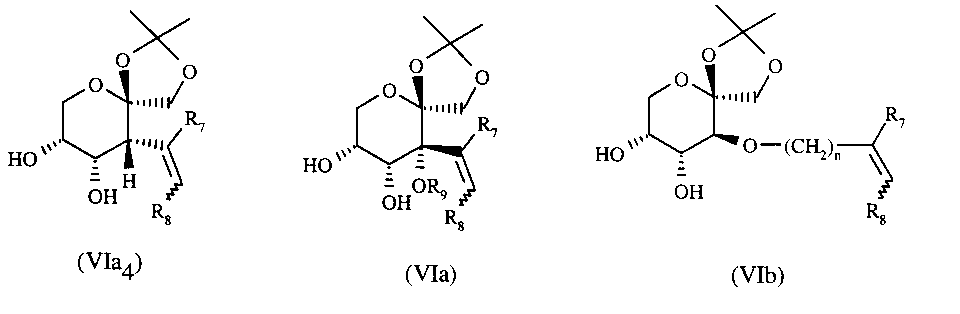 dans lesquelles R₇, R₈, R₉ et n sont tels que définis précédemment,
composés de formules (VIa₄), (VIa) et (VIb) qui peuvent être régiosélectivement substitués en présence d'oxyde de dibutylétain de manière à obtenir respectivement les composés de formules (VIIa4), (VIIa) et (VIIb) : 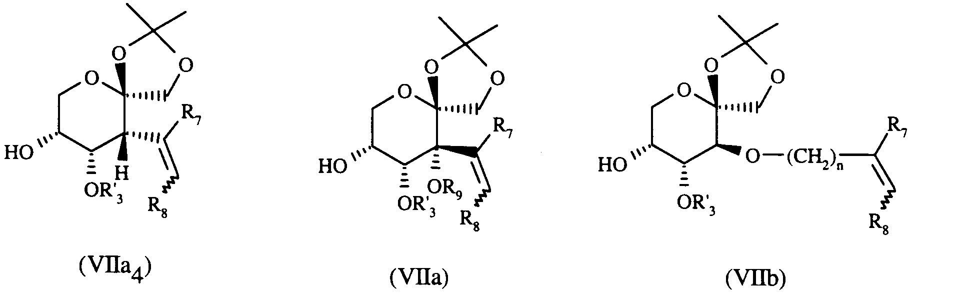 dans lesquelles R₇, R₈, R₉ et n sont tels que définis précédemment et R'₃ est choisi parmi un radical alkyle linéaire ou ramifié comportant de 1 à 6 atomes de carbone et éventuellement substitué, le radical benzyle éventuellement substitué, le radical benzoyle éventuellement substitué, le radical formyle éventuellement substitué, un radical acyle, éventuellement insaturé, linéaire ou ramifié comportant de 1 à 6 atomes de carbone et éventuellement substitué, le radical allyle, et le radical carbamoyle éventuellement mono- ou di-substitué,
l'ensemble des composés de formules (VIa₄) et (VIIa₄), (VIa) et (VIIa) et (VIb) et (VIIb) pouvant éventuellement être soumis à un réactif d'époxydation pour conduire respectivement aux composés de formules (VIIIa₄), (VIIIa) et (VIIIb) : 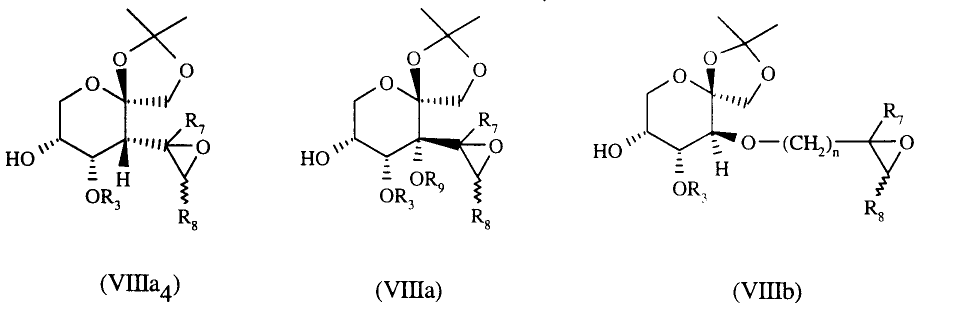 dans lesquelles R₇, R₈, R₉ et n sont tels que définis précédemment, et R₃ est choisi parmi l'hydrogène et le radical R'₃ tel que défini précédemment,
ou bien soumis à hydrogénation catalytique, afin d'obtenir respectivement les composés de formules (VIII'a₄), (VIII'a) et (VIII'b) : 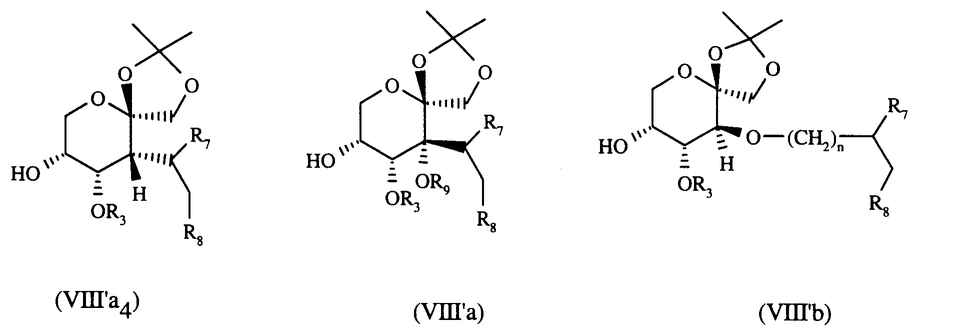 dans lesquelles R₃, R₇, R₈, R₉ et n sont tels que définis précédemment,
l'ensemble des composés de formules (VIa₄), (VIa), (VIb), (VIIa₄), (VIIa), (VIIb), (VIIIa₄), (VIIIa), (VIIIb), (VIII'a₄), (VIII'a) et (VIII'b) formant le composé de formule (VIII) : 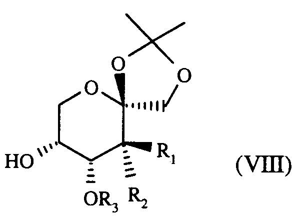 dans laquelle:
**R₁**
- soit représente le radical: 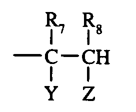 dans lequel Y et Z, soit représentent chacun l'hydrogène, soit forment ensemble une double liaison, soit forment ensemble, avec les atomes de carbone qui les portent, un cycle oxirane, et R₇ et R₈ sont tels que définis précédemment,
et R₂ est choisi parmi l'hydrogène, le radical hydroxy et le radical -OR₉,
- soit le radical : 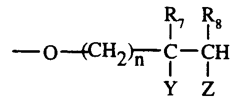 dans lequel n prend les valeurs entières 1 à 4, bornes incluses, et R₇, R₈, Y et Z sont tels que définis précédemment,
et R₂ représente l'hydrogène,
- soit R₁ représente l'hydrogène, et R₂ représente le radical : 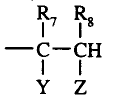 tel que défini ci-dessus et R₃ est tel que précédemment défini,
le composé de formule (VIII) étant ensuite éventuellement soumis:
- à l'action d'un agent alkylant dans des conditions classiques,
- à l'action d'un dérivé isocyanique en présence ou non d'un activateur,
- où à l'action d'un agent carbonylant, le carbonyle diimidazole, conduisant au composé de formule (IXa) : 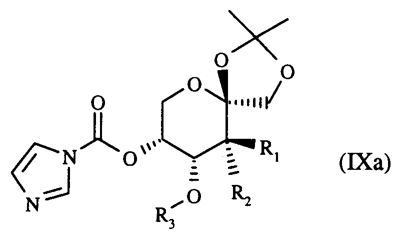
dans laquelle R₁, R₂ et R₃ sont tels que définis précédemment,
lui-même conduisant, sous l'action d'une pipérazine substituée en position 4 par le radical R₉, au composé de formule (IXb) : 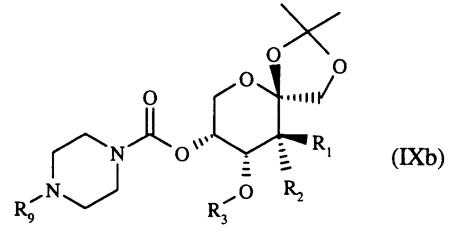 dans laquelle R₁, R₂, R₃ et R₉ sont tels que définis précédemment,
l'ensemble des composés de formules (VIII), (iXa) et (IXb) formant le composé de formule (IX) : 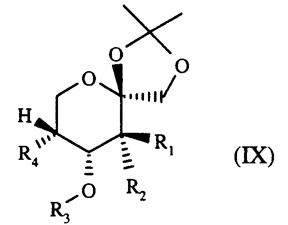 dans laquelle R₁, R₂ et R₃ sont tels que définis précédemment et R₄ est choisi parmi le radical hydroxy, un radical alkoxy linéaire ou ramifié comportant de 1 à 6 atomes de carbone et éventuellement substitué, le radical carbamoyloxy éventuellement mono- ou di-substitué, le radical pipérazinylcarbonyloxy substitué en position 4 par le radical R₉, et le radical imidazolylcarbonyloxy,
le composé de formule (VIII) pouvant également être soumis à l'action d'un agent oxydant dans les conditions utilisées pour l'obtention du composé de formule (III), de manière à obtenir le composé de formule (X) : 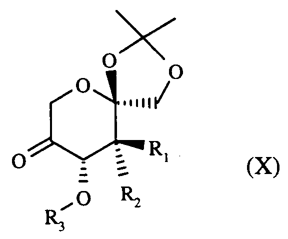 dans laquelle R₁, R₂ et R₃ sont tels que définis précédemment,
l'ensemble des composés de formules (IX) et (X) formant le composé de formule (XI) : 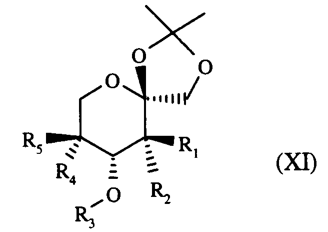 dans laquelle R₁, R₂ et R₃, sont tels que définis précédemment, et R₄ et R₅ sont tels que définis pour la formule (I),
qui peut être:
- **soit** : traité selon des méthodes classiques d'alcoolyse pour former le composé de formule (XII) : 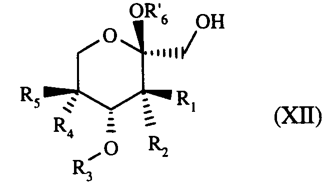 dans laquelle R₁, R₂, R₃, R₄ et R₅ sont tels que définis précédemment et R'₆ est choisi parmi un radical alkyle linéaire ou ramifié comportant de 1 à 6 atomes de carbone et éventuellement substitué, et un radical phénylalkyle dans lequel le groupement phényle est éventuellement substitué et le groupement alkyle, linéaire ou ramifié et éventuellement substitué, comporte de 1 à 6 atomes de carbone,
puis soumis à des réactions classiques de substitution de façon à obtenir le composé de formule (XIII) : 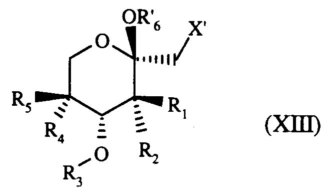 dans laquelle R₁, R₂, R₃, R₄, R₅ et R'₆ sont tels que définis précédemment et X' est choisi parmi un atome d'halogène, un radical phénylsulfonyloxy éventuellement substitué et un radical alkylsulfonyloxy linéaire ou ramifié, éventuellement substitué et comportant de 1 à 6 atomes de carbone,
- **soit :** hydrolysé, en diol de formule (XIV) : 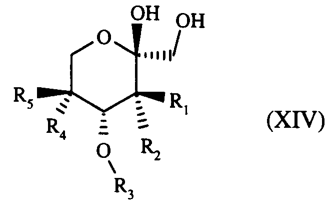 dans laquelle R₁, R₂, R₃, R₄ et R₅ sont tels que définis précédemment,
puis éventuellement soumis à des réactions classiques de substitution de façon à obtenir le composé de formule (XV) : 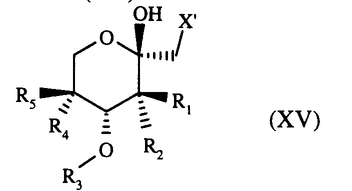 dans laquelle R₁, R₂, R₃, R₄, R₅ et X' sont tels que définis précédemment,
composé de formule (XV) qui, lorsque X' représente l'atome d'iode, peut être transformé, sous l'action d'oxyde d'argent, en époxyde de formule (XVI) : 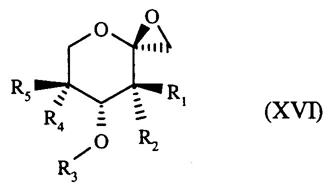 dans laquelle R₁, R₂, R₃, R₄, et R₅ sont tels que définis précédemment,
le composé de formule (XIV) pouvant également être soumis à l'action de N,N-carbonyldiimidazole pour conduire au composé de formule (XVII) : 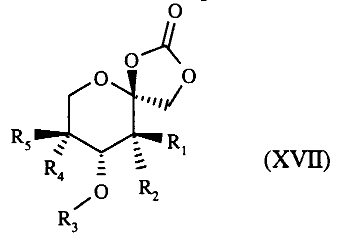 dans laquelle R₁, R₂, R₃, R₄, et R₅ sont tels que définis précédemment,
l'ensemble des composés de formules (XI), (XII), (XIII), (XIV), (XV), (XVI) et (XVII) l'ensemble des composés de formule (I) que l'on purifie le cas échéant par une technique classique de purification et dont on sépare, si on le souhaite, les isomères optiques et géométriques par une technique classique de séparation, et que l'on transforme éventuellement en leurs sels d'addition à un acide, pharmaceutiquement acceptables.

15. Compositions pharmaceutiques contenant comme principe actif au moins un composé selon l'une quelconque des revendications 1 à 13, seul ou en combinaison avec un ou plusieurs véhicules inertes, non toxiques et pharmaceutiquement acceptables.

16. Compositions pharmaceutiques selon la revendication 15 exerçant une activité inhibitrice de l'angiogenèse, et utiles dans le traitement des affections dues ou reliées aux troubles de l'angiogenèse.
